# EUROPÄISCHE PATENTSCHRIFT

(11) **EP 4 117 752 B1**
(45) Veröffentlichungstag und Bekanntmachung des Hinweises auf die Patenterteilung: **10.12.2025**
(21) Anmeldenummer: 21718486.0
(22) Anmeldetag: 04.03.2021
(51) Int. Cl.: A61M 5/158, A61M 25/06

(54) **KANÜLENANORDNUNG, INSBESONDERE ZUR ENTNAHME VON FLÜSSIGKEIT AUS EINEM KÖRPER**
CANNULA ARRANGEMENT, IN PARTICULAR FOR WITHDRAWAL OF LIQUIDS FROM A BODY
ARRANGEMENT D'UNE CANULE, EN PARTICULIER POUR LE PRELEVEMENT DES LIQUIDES D'UN CORPS

(30) Priorität: 09.03.2020 AT 501882020
(43) Veröffentlichungstag der Anmeldung: 18.01.2023
(73) Patentinhaber: Greiner Bio-One GmbH, 4550 Kremsmünster (AT)
(72) Erfinder: RATHNER, Christian, 4642 Sattledt (AT); STRASSER, Gerhard, 4550 Kremsmünster (AT); KOFLER, Georg, 4565 Inzersdorf im Kremstal (AT)
(74) Vertreter: Burger, Hannes
(86) Internationale Anmeldenummer: PCT/AT2021/060072
(87) Internationale Veröffentlichungsnummer: WO 2021/179026

(56) Entgegenhaltungen:
- EP-A1- 2 075 020
- WO-A1-2009/021263
- WO-A1-2016/007438
- WO-A1-2016/007981
- CN-U- 209 645 575
- JP-U- S5 578 344

## Beschreibung

Die Erfindung betrifft eine Kanülenanordnung, insbesondere eine Sicherheits-Kanülenanordnung, welche zur Entnahme von Flüssigkeit aus einem Körper und/oder zur Zufuhr einer Flüssigkeit in einen Körper dient.

Die WO 2016/007981 A1 der gleichen Anmelderin beschreibt eine Sicherheitsnadelanordnung umfassend einen Basiskörper, einen Kanülenträger und eine Kanüle, welche mit ihrem proximalen Ende an einem distalen Ende des Kanülenträgers gehalten ist. Weiters umfasst die Sicherheitsnadelanordnung noch ein als Feder ausgebildetes Stellelement, welches zwischen dem Basiskörper und dem Kanülenträger wirkend angeordnet ist, sowie eine Verriegelungsvorrichtung mit mehreren ersten und zweiten miteinander in Eingriff stehenden Verriegelungselementen. Die ersten Verriegelungselemente sind im Bereich eines proximalen Endes des Basiskörpers angeordnet und durch einander diametral gegenüberliegend angeordnete Vorsprünge gebildet. Die zweiten Verriegelungselemente sind im Bereich eines proximalen Endes des Kanülenträgers angeordnet und durch einander diametral gegenüberliegend angeordnete Ausnehmungen gebildet. Diese Sicherheitsnadelanordnung hat sich in der Praxis gut bewährt, und dient für die vorliegende Erfindung als Basis. Nachteilig sind die mehrfach erforderlichen Werkzeuge, was zu höheren Stückkosten führt.

Die US 2007/0016148 A1 beschreibt eine weitere gattungsgemäß ausgebildete Sicherheitsnadelanordnung umfassend einen Basiskörper mit einem distalen Ende und einem proximalen Ende, wobei sich zwischen dem distalen Ende des Basiskörpers und dem proximalen Ende des Basiskörpers in diesem eine Durchgangsöffnung erstreckt. Weiters umfasst die Sicherheitsnadelanordnung einen Kanülenträger mit einem distalen Ende und einem proximalen Ende, wobei sich zwischen den beiden Enden des Kanülenträgers eine Durchströmöffnung erstreckt. Der Kanülenträger ist in der Durchgangsöffnung des Basiskörpers in Axialrichtung verstellbar aufgenommen. Eine Kanüle weist ein distales Ende und ein proximales Ende auf, wobei sich zwischen den beiden Enden der Kanüle innerhalb dieser ein Strömungskanal erstreckt, und das proximale Ende der Kanüle an dem distalen Ende des Kanülenträgers gehalten ist. Ein Stellelement ist zwischen dem Basiskörper und dem Kanülenträger wirkend angeordnet, wobei das Stellelement den Kanülenträger mitsamt der daran gehaltenen Kanüle von einer ersten Stellung, bei welcher die Kanüle über das distale Ende des Basiskörpers vorragt, in eine zweite Stellung selbsttätig verlagert, bei welcher zumindest das distale Ende der Kanüle vom Basiskörper abgedeckt ist. Eine Verriegelungsvorrichtung weist mehrere erste und zweite Verriegelungselemente auf. Durch die Verriegelungsvorrichtung ist bei einer sich in gegenseitigem Eingriff befindlichen Position der ersten und zweiten Verriegelungselemente die erste relative Stellung des Kanülenträgers mitsamt der Kanüle bezüglich des Basiskörpers festlegt. Die ersten Verriegelungselemente sind im Bereich des proximalen Endes des Basiskörpers und die zweiten Verriegelungselemente im Bereich des proximalen Endes des Kanülenträgers angeordnet. Nachteilig dabei ist, dass nach dem Entriegeln der Verriegelungsvorrichtung und dem damit verbundenen Halten des Kanülenträgers vom Stellelement eine Druckkraft in Axialrichtung auf den Basiskörper ausgeübt wird und dadurch der Basiskörper in Richtung auf die Einstichstelle gedrückt wird. Dies führt zu einem zusätzlichen auf den Körper ausgeübten Schmerzreiz.

Die JP S55 78344U, insbesondere die Fig. 2 dieser Schrift, zeigt einen Nadelhalter mit einer Nadelaufnahmebohrung, welche Nadelaufnahmebohrung eine abgestufte Ausbildung mit zueinander unterschiedlichen Bohrungsdurchmessern für jeweils unterschiedliche Kanülendurchmesser aufweist.

Aufgabe der vorliegenden Erfindung war es, die Nachteile des Standes der Technik zu überwinden und eine Kanülenanordnung zur Verfügung zu stellen, welche eine einfachere Herstellung bei universelleren Kombinationsmöglichkeiten aufweist.

Diese Aufgabe wird durch eine Kanülenanordnung, insbesondere durch eine Sicherheits-Kanülenanordnung, gemäß den Ansprüchen gelöst.

Die erfindungsgemäße Kanülenanordnung wird in der Medizintechnik eingesetzt, um mittels dieser eine Flüssigkeit aus dem Körper entnehmen zu können und/oder eine Flüssigkeit dem Körper zuführen zu können.

Die Kanülenanordnung umfasst
- einen Kanülenträger mit einem distalen Ende und einem proximalen Ende, wobei sich innerhalb des Kanülenträgers zwischen seinem distalen Ende und seinem proximalen Ende ein Durchströmkanal erstreckt, und der Durchströmkanal einen distalen Durchströmkanal-Endabschnitt und einen proximalen Durchströmkanal-Endabschnitt definiert,
- eine Kanüle mit einem distalen Ende und einem proximalen Ende, wobei sich innerhalb der Kanüle zwischen ihrem distalen Ende und ihrem proximalen Ende ein Strömungskanal erstreckt, und
- wobei das proximale Ende der Kanüle im distalen Durchströmkanal-Endabschnitt des Kanülenträgers aufgenommen und am Kanülenträger gehalten ist, insbesondere mit diesem verklebt ist, und die Kanüle und der Kanülenträger eine Längsachse definieren, wobei weiters noch vorgesehen ist
- dass im Axialschnitt gesehen der distale Durchströmkanal-Endabschnitt ausgehend vom distalen Ende des Kanülenträgers in Richtung auf das proximale Ende des Kanülenträgers eine abgestuft ausgebildete Querschnittsform mit einer ersten Querschnittsabmessung und zumindest einer weiteren Querschnittsabmessung aufweist,
- dass jede der Querschnittsabmessungen einen eigenen Aufnahmeabschnitt für die jeweilige darin aufzunehmende Kanüle definiert,
- dass die Querschnittsabmessung jedes Aufnahmeabschnitts ausgehend vom distalen Ende des Kanülenträgers in Richtung auf das proximale Ende des Kanülenträgers jeweils geringer ist als die Querschnittsabmessung des unmittelbar in Axialrichtung vorgeordneten Aufnahmeabschnitts, und
- dass jede der zueinander unterschiedlichen Querschnittsabmessungen der Aufnahmeabschnitte derart ausgebildet ist, um jeweils jene Kanüle mit einer dazu korrespondierenden Kanülen-Querschnittsabmessung aufnehmen zu können.

Der dadurch erzielte Vorteil liegt darin, dass so durch die mehrfache Ausbildung von zueinander unterschiedlichen Aufnahmeabschnitten in ein und denselben Kanülenträger die jeweils vorbestimmte Kanüle mit der zum jeweiligen Aufnahmeabschnitt korrespondierenden Kanülen-Querschnittsabmessung aufzunehmen und auch daran zu befestigen. Die einzelnen Aufnahmeabschnitte sind in ihren jeweiligen Querschnittsabmessungen an die jeweilige Kanülen-Querschnittsabmessung abgestimmt. Dadurch wird für jede Kanüle ein eigener dazu abgestimmter Aufnahmeabschnitt bereitgestellt, welcher exakt an die Kanüle angepasst ist. Je nach Anzahl der gewählten einzelnen Aufnahmeabschnitte ist mit ein und demselben Werkzeug (Spritzgusswerkzeug) die Herstellung nur eines jeweils gleichen Kanülenträgers erforderlich, da je nach Bedarf die Kanüle mit der vorbestimmten Querschnittsabmessung bei der Montage in den dafür vorgesehenen Aufnahmeabschnitt eingesetzt und befestigt wird.

Weiters ist ein Basiskörper mit einem distalen Ende und einem proximalen Ende vorgesehen, wobei sich zwischen dem distalen Ende des Basiskörpers und dem proximalen Ende des Basiskörpers in diesem eine Durchgangsöffnung erstreckt und dass zumindest der Kanülenträger mit der daran gehaltenen Kanüle in der Durchgangsöffnung aufgenommen sowie in der Durchgangsöffnung in Axialrichtung verstellbar ist. Damit wird es möglich, eine Sicherheits-Kanülenanordnung bereitzustellen, in welcher der Kanülenträger mit seiner daran gehaltenen Kanüle aufgenommen und geführt werden kann.

Erfindungsgemäß ist weiters eine Flügelanordnung mit einem rohrförmig ausgebildeten Haltekörper und vom Haltekörper beidseits abstehenden Flügel vorgesehen, wobei die Flügel eine einem Patienten zuwendbare Anlageseite definieren und dass weiters eine Kopplungseinheit zwischen dem Haltekörper und dem Basiskörper angeordnet oder ausgebildet ist und mittels der Kopplungseinheit die Flügelanordnung in einer Kopplungsstellung am Basiskörper gehalten ist. Damit kann die Betätigung und Handhabung der Sicherheits-Kanülenanordnung für den Benutzer erleichtert werden. Weiters wird damit aber auch eine bessere und positionsgenauere Anbringung an einem Körper ermöglicht.

Erfindungsgemäß ist weiters ein Sichtfenster im rohrförmig ausgebildeten Haltekörper ausgebildet, welches Sichtfenster an einer von der Anlageseite abgewendeten Seite angeordnet ist und dass in der Kopplungsstellung ein vom Basiskörper in radialer Richtung abstehender Ansatz in das Sichtfenster hineinragt. Damit kann für den Benutzer bei der Wahl eines durchscheinenden oder durchsichtigen Werkstoffs im Bereich des Kanülenendes die Möglichkeit geschaffen, den Ansticherfolg optisch einfach erkennbar zu machen.

Weiters kann es vorteilhaft sein, wenn die Aufnahmeabschnitte jeweils mit einem hohlzylinderförmigen Querschnitt ausgebildet sind. Damit kann eine gute und umfangsmäßig gleichmäßigere Verteilung des Klebemittels zur Befestigung der Kanüle am Kanülenträger erzielt werden.

Eine andere Ausführungsform zeichnet sich dadurch aus, dass mehrere, insbesondere drei, in Axialrichtung hintereinander ausgebildete Aufnahmeabschnitte vorgesehen sind. Je nach gewählter Anzahl der hintereinander befindlichen Aufnahmeabschnitte kann so ein noch universellerer Einsatz bei gleichzeitiger Kostenreduktion erzielt werden.

Eine weitere mögliche Ausführungsform hat die Merkmale, dass jeder der Aufnahmeabschnitte an seinem proximalen Ende jeweils einen Axialanschlag für das proximale Ende der jeweiligen darin aufgenommen Kanüle bildet. Weiters kann noch vorgesehen sein, dass das jeweilige proximale Ende der Kanüle an jenem dazu korrespondierenden Axialanschlag des jeweiligen Aufnahmeabschnitts daran in Richtung auf das proximale Ende des Kanülenträgers anliegend abgestützt ist. Damit kann für jede unterschiedliche Kanülen-Querschnittsabmessung eine gute Positioniergenauigkeit in jeweiligen Aufnahmeabschnitt , insbesondere in Axialrichtung, erzielt werden.

Eine andere Ausführungsform zeichnet sich dadurch aus, dass der Kanülenträger von einem weiblichen Luer-Kopplungsteil oder einer Bauteilkomponente einer Sicherheits-Kanülenanordnung gebildet ist. Damit können die unterschiedlichsten medizinischen Tragkörper für unterschiedlichste Kanülenabmessungen (Durchmesser) bereitgestellt werden, wobei für jede Type mit nur einer Ausführung oder einer zusätzlichen weiteren Ausführungsform das Auslangen gefunden werden kann.

Weiters kann es vorteilhaft sein, wenn weiters ein selbsttätig wirkendes Stellelement vorgesehen ist, welches Stellelement zwischen dem Basiskörper und dem Kanülenträger wirkend angeordnet ist, und das Stellelement den Kanülenträger mitsamt der daran gehaltenen Kanüle von einer ersten Stellung, bei welcher ersten Stellung die Kanüle über das distale Ende des Basiskörpers vorragt, in eine zweite Stellung verlagert, bei welcher zweiten Stellung zumindest das distale Ende der Kanüle vom Basiskörper abgedeckt ist. Damit kann eine selbsttätige relative Verlagerung des Kanülenträger mitsamt der daran gehaltenen Kanüle relativ bezüglich des Basiskörper erzielt werden. Dies erfolgt zumeist erst nach Freigabe von gegenseitig in Eingriff stehenden Halteelementen.

Eine andere alternative Ausführungsform zeichnet sich dadurch aus, dass weiters eine Haltevorrichtung mit mehreren ersten Halteelementen und mehreren zweiten Halteelementen vorgesehen ist, welche Haltevorrichtung bei der sich in gegenseitigem Eingriff befindlichen Position der ersten und zweiten Halteelemente die erste Stellung des Kanülenträgers bezüglich des Basiskörpers festlegt, und dass die ersten Halteelemente im Bereich des proximalen Endes des Basiskörpers und die zweiten Halteelemente im Bereich des proximalen Endes des Kanülenträgers angeordnet sind. Damit wird eine definierte Ausgangsstellung bzw. Einsatzstellung ermöglicht.

Eine weitere mögliche und gegebenenfalls alternative Ausführungsform hat die Merkmale, dass weiters eine Arretiervorrichtung mit mehreren ersten und zweiten Arretierelementen vorgesehen ist, welche Arretiervorrichtung bei der sich in gegenseitigem Eingriff befindlichen Position der ersten und zweiten Arretierelemente die zweite Stellung des Kanülenträgers bezüglich des Basiskörpers festlegt, und dass die ersten Arretierelemente im Bereich des proximalen Endes des Basiskörpers und die zweiten Arretierelemente im Bereich des distalen Endes des Kanülenträgers angeordnet sind. Damit kann eine Wiederverwendung und/oder auch eine Stichverletzung nach dem Verbringen des Kanülenträgers in seine zweite Stellung verhindert werden.

Eine weitere Ausbildung sieht vor, dass zumindest eine Entlastungsvertiefung im Kanülenträger angeordnet oder ausgebildet ist, und dass bei sich in der ersten Stellung befindlichem Kanülenträger die ersten Arretierelemente in ihrer unverformten Stellung in die zumindest eine Entlastungsvertiefung hineinragen. Damit kann während des Lagervorgangs eine unverformte Ausgangsstellung der ersten Arretierelemente erzielt werden. Weiters kann ein ansonsten bei einer vorverformten Lagerstellung eintretender innerer Spannungsabbau verhindert werden.

Weiters kann es vorteilhaft sein, wenn die Kopplungseinheit weiters eine Verdreh-Sicherungsvorrichtung mit zumindest einem ersten Verdreh-Sicherungselement am oder im Basiskörper und zumindest ein damit zusammenwirkendes zweites Verdreh-Sicherungselement im oder am Haltekörper umfasst. Durch das Vorsehen einer eigenen Verdreh-Sicherungsvorrichtung kann eine eindeutige und verdrehfeste Halterung der Flügelanordnung am Basiskörper erzielt werden.

Eine andere Ausführungsform zeichnet sich dadurch aus, dass das zumindest eine erste Verdreh-Sicherungselement als Steg und das zumindest eine zweite Verdreh-Sicherungselement als Nut ausgebildet ist und dass die Verdreh-Sicherungselemente eine parallele Längsausrichtung bezüglich der Längsachse aufweisen. Damit kann eine formschlüssige und exakte Positionierung der in Kopplungseingriff stehenden Bauteilkomponenten erzielt werden.

Eine weitere mögliche Ausführungsform hat die Merkmale, dass die Kopplungseinheit weiters eine Axial-Sicherungsvorrichtung mit zumindest einem ersten Axial-Sicherungselement am oder im Basiskörper und zumindest ein damit zusammenwirkendes zweites Axial-Sicherungselement im oder am Haltekörper umfasst. Weiters kann noch vorgesehen sein, dass die Axial-Sicherungselemente im Axialschnitt gesehen in etwa mit einem Dreieckquerschnitt ausgebildet sind, wobei eine erste Begrenzungslinie des Dreieckquerschnitts in axialer Aufschubrichtung der Flügelanordnung auf den Basiskörper gesehen eine auf die von der Längsachse abgewendete Seite ansteigend ausgebildete Rampe definiert und eine zweite Begrenzungslinie des Dreieckquerschnitts in einer Normalebene bezüglich der Längsachse verlaufend ausgerichtet ist. Damit kann eine Halterung der Flügelanordnung am Basiskörper in zur Aufschubrichtung in einer dazu entgegen gesetzten Richtung eine Lösebewegung verhindert werden.

Eine andere Ausführungsform zeichnet sich dadurch aus, dass weiters eine insbesondere rohrförmig ausgebildete Schutzhülle vorgesehen ist und die Schutzhülle am distalen Ende des Basiskörpers angeordnet und an diesem abnehmbar gehalten ist, wobei von der Schutzhülle die in der ersten Stellung vom Basiskörper in distaler Richtung vorragende Kanüle abgedeckt ist. Damit kann vor dem Erstgebrauch und vor der Abnahme der Schutzhülle vom distalen Ende des Basiskörpers eine Stichverletzung verhindert werden. Die Schutzhülle wird bevorzugt von einer umlaufenden Hüllenwand gebildet und weist eine vordefinierte Axiallänge auf.

Eine weitere bevorzugte Ausführungsform ist dadurch gekennzeichnet, dass zumindest zwei erste Längsrippen und zumindest zwei zweite Längsrippen an einer der Längsachse zugewendeten Innenfläche der Schutzhülle über den Umfang verteilt angeordnet oder ausgebildet sind, und dass in Umfangsrichtung jeweils abwechseln eine erste Längsrippe und eine zweite Längsrippe angeordnet sind. Durch die Wahl der ersten und zweiten Längsrippen kann so über den Innenumfang gesehen eine vorbestimmte Ausbildung und Anordnung ermöglicht werden.

Weiters kann es vorteilhaft sein, wenn bei unverformtem Querschnitt der Schutzhülle erste Enden der ersten Längsrippen in einer ersten Radialdistanz vor der Längsachse enden und zweite Enden der zweiten Längsrippen in einer zweiten Radialdistanz vor der Längsachse enden, wobei die erste Radialdistanz größer ausgebildet ist als die zweite Radialdistanz. Durch die unterschiedlich große Distanzierung der ersten Enden und der zweiten Enden von der gemeinsamen Längsachse kann so bei auf den Basiskörper aufgesetzter Schutzhülle ein besserer Toleranzausgleich erzielt werden. Damit kann im Bereich jener Längsrippen mit der größeren radialen Distanzierung von deren Enden von der Längsachse die radiale Vorspannkraft etwas reduziert werden und damit eine geringere Aufweitung der Hüllenwand in diesem Umfangsabschnitt erzielt werden.

Eine andere alternative Ausführungsform zeichnet sich dadurch aus, dass die ersten Enden der ersten Längsrippen auf einer ersten Kreisbahn und die zweiten Enden der zweiten Längsrippen auf einer zweiten Kreisbahn liegend angeordnet sind und die beiden Kreisbahnen konzentrisch bezüglich der Längsachse angeordnet sind. Damit kann über den Umfang gesehen, eine gleichmäßige radiale Vorspannkraft von der Schutzhülle auf den Halteansatz des Basiskörpers aufgebaut werden.

Eine weitere mögliche und gegebenenfalls alternative Ausführungsform hat die Merkmale, dass ein Differenzwert der erste Radialdistanz abzüglich der zweiten Radialdistanz aus einem Differenz-Wertebereich stammt, dessen untere Grenze 0,01 mm, bevorzugt 0,04 mm, und dessen obere Grenze 0,1 mm, bevorzugt 0,06 mm, beträgt. Damit kann je nach gewähltem Differenzwert der mögliche Toleranzausgleich festgelegt werden. Darüber hinaus kann so aber auch die Haltekraft am Basiskörper und die erforderliche axiale Abzugskraft in gewissen Grenzen festgelegt werden.

Eine weitere Ausbildung sieht vor, dass die Gesamtanzahl der ersten Längsrippen und der zweiten Längsrippen eine gerade Anzahl ist und aus einer Gesamtanzahl von vier, sechs, acht oder zehn ausgewählt ist. Damit kann die Schutzhülle einfach an unterschiedliche Einsatzbedingungen und Abmessungen abgestimmt werden.

Eine andere Ausführungsform zeichnet sich dadurch aus, dass die ersten Längsrippen und/oder zweiten Längsrippen im Radialschnitt gesehen jeweils eine Wellenform aufweisen. Damit können sprunghafte Übergänge in der Wandstärke vermieden werden.

Zum besseren Verständnis der Erfindung wird diese anhand der nachfolgenden Figuren näher erläutert.

Es zeigen jeweils in stark vereinfachter, schematischer Darstellung:
- Fig. 1: eine Kanülenanordnung in ihrer unbenutzten Einsatzstellung, in schaubildlicher Darstellung;
- Fig. 2: die Kanülenanordnung nach Fig. 1, jedoch in ihrer Schutzstellung der Kanüle, in schaubildlicher Darstellung, teilweise geschnitten;
- Fig. 3: die Kanülenanordnung nach den Fig. 1 und 2 in einer Art Explosionsdarstellung mit voneinander distanzierten Einzelkomponenten;
- Fig. 4: den Kanülenträger mit der daran gehaltenen Kanüle, im Axialschnitt und vergrößerter Darstellung;
- Fig. 5: den Basiskörper der Kanülenanordnung nach den Fig. 1 bis 4, in schaubildlicher Darstellung;
- Fig. 6: die Flügelanordnung der Kanülenanordnung nach den Fig. 1 bis 5, in schaubildlicher Darstellung;
- Fig. 7: ein möglicher Querschnitt der Schutzhülle der Kanülenanordnung, in vergrößerter Darstellung;
- Fig. 8: einen anderen, als weiblicher Luer-Kopplungsteil ausgebildeter Kanülenträger mit daran gehaltener Kanüle, im Axialschnitt.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

Der Begriff "insbesondere" wird nachfolgend so verstanden, dass es sich dabei um eine mögliche speziellere Ausbildung oder nähere Spezifizierung eines Gegenstands oder eines Verfahrensschritts handeln kann, aber nicht unbedingt eine zwingende, bevorzugte Ausführungsform desselben oder eine zwingende Vorgehensweise darstellen muss.

Es werden nachfolgend die Begriffe "distal" und "proximal" verwendet. Die Lageangabe oder Richtungsangabe mit dem Begriff "distal" ist so auszulegen, dass dieses Ende oder dieser Endabschnitt des jeweiligen Gegenstandes vom Anwender desselben abgewendet und einem Patienten zuwendbar ist. Die weitere Lageangabe oder Richtungsangabe mit dem Begriff "proximal" ist so auszulegen, dass dieses Ende oder dieser Endabschnitt des jeweiligen Gegenstandes dem Anwender desselben zugewendet und vom Patienten abgewendet ist.

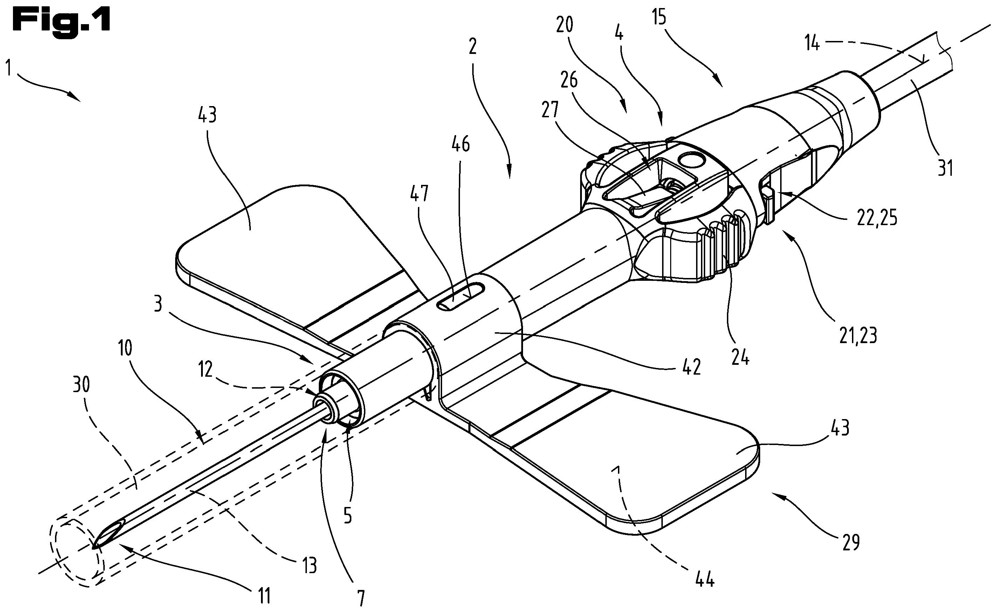

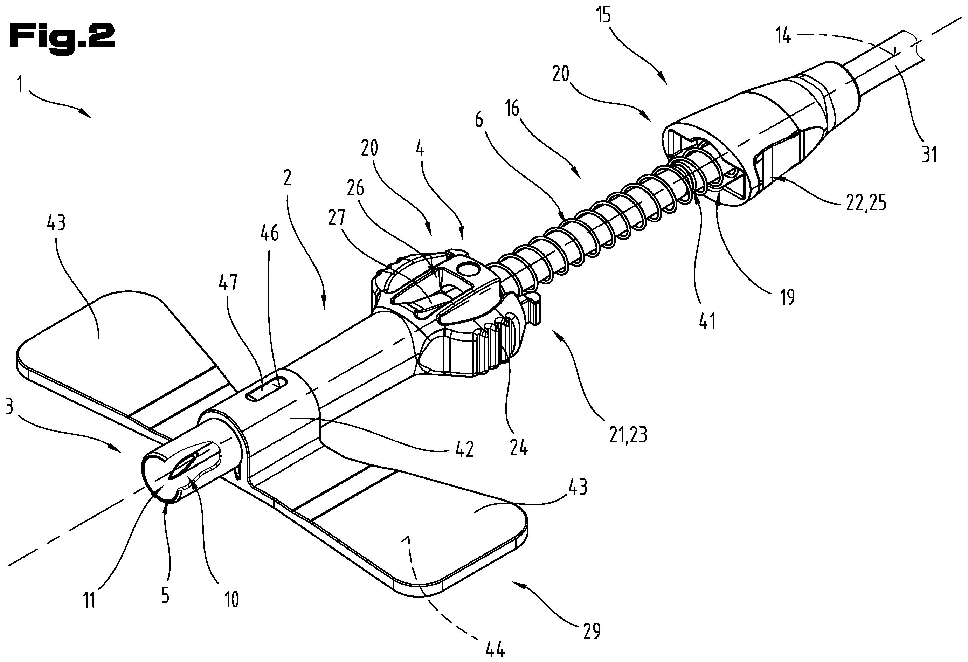

In den Fig. 1 bis 7 ist eine Kanülenanordnung 1 gezeigt, welche grundsätzlich in der Medizintechnik Verwendung findet. Derartige Kanülenanordnungen 1 können auch als Sicherheits-Kanülenanordnung oder als Sicherheitsnadelanordnung bezeichnet werden. Die Kanülenanordnung 1 kann beispielsweise zur Entnahme von Flüssigkeit, insbesondere Blut oder eine andere Körperflüssigkeit, aus einem Körper und/oder zum Zuführen von Flüssigkeit in den Körper eingesetzt werden. Die gezeigte Kanülenanordnung 1 kann auch als sogenannter "Butterfly" bezeichnet werden, welcher üblicher Weise während der bestimmungsgemäßen Benutzung am Körper gehalten wird. Dies kann z.B. mittels eines Klebebandes erfolgen, wie dies hinlänglich bekannt ist. Die Fig. 1 und 2 zeigen die Kanülenanordnung 1 in deren zusammengebauten Zustand, jedoch in zueinander unterschiedlichen Stellungen von deren einzelnen Bauteilkomponenten zueinander.

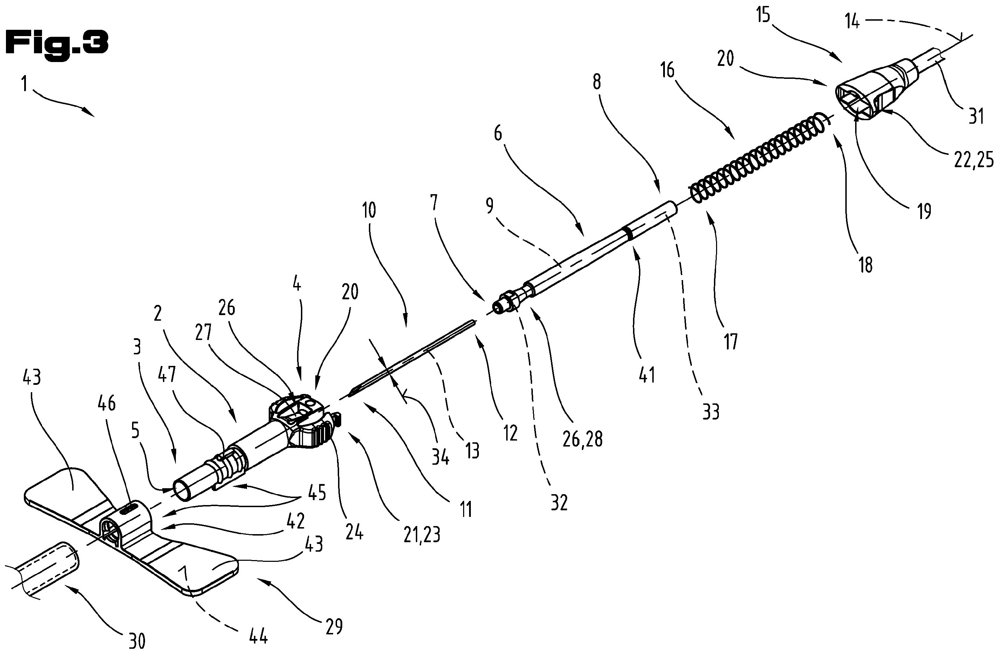

In der Fig. 3 sind die einzelnen Bauteile bzw. Bauteilkomponenten der Kanülenanordnung 1 in einer voneinander getrennten Anordnung gezeigt, um deren Ausbildung besser ersehen und beschreiben zu können.

Im vorliegenden Ausführungsbeispiel umfasst die Kanülenanordnung 1 einen Basiskörper 2 mit einem distalen Ende 3 und einem proximalen Ende 4 umfassen. Bevorzugt ist der Basiskörper 2 rohrförmig, insbesondere hohlzylinderförmig, ausgebildet und weist in Richtung seiner Axialerstreckung eine sich zwischen dem distalen Ende 3 des Basiskörpers 2 und dem proximalen Ende 4 des Basiskörper 2 erstreckende Durchgangsöffnung 5 auf.

Weiters kann die Sicherheitsnadelanordnung 1 auch noch einen Kanülenträger 6 umfassen, welcher seinerseits ein distales Ende 7 sowie ein in Axialrichtung davon distanziertes proximales Ende 8 aufweist. Im Kanülenträger 6 erstreckt sich zwischen dem distalen Ende 7 und dem proximalen Ende 8 ein Durchströmkanal 9. Des Weiteren ist der Kanülenträger 6 derart ausgebildet, dass dieser in der Durchgangsöffnung 5 des Basiskörpers 2 aufgenommen und in Axialrichtung darin verstellbar geführt ist.

Des Weiteren kann die Kanülenanordnung 1 auch noch eine Kanüle 10 umfassen, welche ihrerseits ein distales Ende 11 sowie ein in Axialrichtung davon distanziertes proximales Ende 12 aufweist. Innerhalb der Kanüle 10 erstreckt sich zwischen dem distalen Ende 11 und dem proximalen Ende 12 ein Strömungskanal 13. Weiters kann das proximale Ende 12 der Kanüle 10 an dem distalen Ende 7 des Kanülenträgers 6 gehalten, insbesondere daran feststehend befestigt sein. Die Verbindung des proximalen Endes 12 der Kanüle 10 mit dem Kanülenträger 6 kann z.B. durch eine zumindest luftdichte Klebeverbindung erfolgen. Es wäre unabhängig davon aber auch noch möglich, anstatt der Klebeverbindung oder zusätzlich zu dieser, einen Presssitz auszubilden. Dazu ist ein entsprechender Abmessungsunterschied zwischen dem Endbereich der Kanüle 10 und der Aufnahmeöffnung im Kanülenträger 6 auszubilden. Der Kanülenträger 6 und die an diesem gehaltene Kanüle 10 sind bevorzugt koaxial zueinander ausgerichtet und definieren gemeinsam eine Längsachse 14.

Damit kann ausgehend vom distalen Ende 11 der Kanüle 10 ein Durchfluss an Flüssigkeit durch den Strömungskanal 13 und weiters durch den Durchströmkanal 9 im Kanülenträger 6 oder auch in dazu entgegengesetzter Richtung erzielt werden.

Weiters kann der Kanülenträger 6 im Bereich seines proximalen Endes 8 auch noch einen zusätzlichen Halteansatz 15 aufweisen. Der Halteansatz 15 kann entweder durch einen eigenen Bauteil gebildet sein oder aber auch integraler Bestandteil des Kanülenträgers 6 sein. Handelt es sich beispielsweise, wie im vorliegenden Ausführungsbeispiel, um jeweils eigene Bauteile, kann sich das proximale Ende 8 des Kanülenträgers 6 in Axialrichtung gesehen in den Halteansatz 15 hinein erstrecken und ist dort mit diesem verbunden. Auch hier kann die gegenseitige Halterung bzw. Verbindung zwischen dem proximalen Ende 8 des Kanülenträgers 6 und dem Halteansatz 15 kann z.B. durch eine zumindest luftdichte Klebeverbindung erfolgen. Es wäre unabhängig davon aber auch noch möglich, anstatt der Klebeverbindung oder zusätzlich zu dieser, einen Presssitz auszubilden. Dazu ist ein entsprechender Abmessungsunterschied zwischen dem Endbereich des Kanülenträgers 6 und dem Halteansatz 15 auszubilden.

Zur selbsttätigen Verlagerung des Kanülenträgers 6 relativ bezüglich des Basiskörpers 2 kann die Kanülenanordnung 1 auch noch ein eigenes Stellelement 16 umfassen, welches im Einbauzustand zwischen dem Basiskörper 2 und dem Kanülenträger 6 wirkend angeordnet ist. Im vorliegenden Ausführungsbeispiel ist das Stellelement 16 durch eine Spiraldruckfeder gebildet, welche sich an der Außenseite des Kanülenträgers 6 erstreckend angeordnet ist. Ein dem Basiskörper 2 zugewendetes distales Stellelementende 17 ist im Bereich des proximalen Endes 4 des Basiskörpers 2 an diesem abgestützt. Das Stellelement 16 kann sich auch noch in Axialrichtung über eine Teillänge in den Basiskörper 2 hinein erstrecken.

Im vorliegenden Ausführungsbeispiel ist das weitere in Axialrichtung davon distanzierte proximale Stellelementende 18 des Stellelements 16 am Halteansatz 15 abgestützt. Um in der zusammengeschobenen Einsatzposition bzw. vorgespannten Stellung des Stellelementes 16 ausreichend Platz dafür zu haben, kann es vorteilhaft sein, wenn sich das Stellelement 16 über eine Teillänge auf der dem Basiskörper 2 zugewendeten Seite in den Halteansatz 15 hinein erstrecken kann. Dazu ist im Halteansatz 15 auf seiner dem Basiskörper 2 zugewendeten Seite eine sich über eine Teillänge zwischen dem Kanülenträger 6 und dem Halteansatz 15 erstreckende Aufnahmeöffnung 19 zur Aufnahme des Stellelements 16 angeordnet bzw. vorgesehen. Die Querschnittsform der Aufnahmeöffnung 19 ist dabei bevorzugt an die Abmessungen des Stellelements 16 anzupassen. Im vorliegenden Ausführungsbeispiel ist die Aufnahmeöffnung 19 beispielsweise rohrförmig bzw. hohlzylinderförmig ausgebildet.

In der Fig. 1 ist die erste relative Stellung des Kanülenträgers 6 mitsamt der daran gehaltenen Kanüle 10 gezeigt. In dieser ersten Stellung ragt die Kanüle 10 über das distale Ende 3 des Basiskörpers 2 vor.

In der Fig. 2 ist die sogenannte Schutzstellung gezeigt, welche hier als zweite Stellung bezeichnet wird. In dieser zweiten Stellung ist zumindest das distale Ende 11 der Kanüle 10 vom Basiskörper 2 abgedeckt, um so Stichverletzungen durch die gebrauchte Kanüle 10 zu vermeiden. Die Verlagerung, insbesondere die Axialverstellung, des Kanülenträgers 6 mitsamt der daran gehaltenen Kanüle 10 erfolgt durch das zuvor beschriebene Stellelement 16 selbsttätig. Wird der Basiskörper 2 als ortsfest positioniert angesehen, erfolgt die relative Verlagerung des Kanülenträgers 6 mitsamt der Kanüle 10 in proximaler Richtung.

Um den Kanülenträger 6 mitsamt der daran gehaltenen Kanüle 10 in der ersten Stellung relativ bezüglich des Basiskörpers 2 verrastet bzw. verriegelt halten zu können, ist eine eigene Haltevorrichtung 20 vorgesehen, welche ebenfalls Bestandteil der Kanülenanordnung 1 sein kann. Die Haltevorrichtung 20 umfasst bei diesem Ausführungsbeispiel mehrere erste Halteelement 21 sowie mehrere zweite Verriegelungselemente 22. In der Halte- bzw. Verriegelungsstellung der Haltevorrichtung 20 wird durch die in Eingriff befindlichen ersten Halteelemente 21 und zweiten Halteelemente 22 die erste relative Stellung des Kanülenträgers 6 mitsamt der Kanüle 10 bezüglich des Basiskörpers 2 festgelegt.

Die ersten Halteelemente 21 sind im vorliegenden Ausführungsbeispiel durch einander diametral gegenüberliegend angeordnete Vorsprünge 23 gebildet und sind im Bereich des proximalen Endes 4 des Basiskörpers 2 an diesem angeordnet oder ausgebildet. Weiters können die Vorsprünge 23 jeweils an einem in Radialrichtung verstellbaren Haltearm 24 angeordnet sein.

Die zweiten Halteelemente 22 sind im vorliegenden Ausführungsbeispiel durch Ausnehmungen 25 gebildet, welche ebenfalls einander diametral gegenüberliegend angeordnet sind, um einen Eingriff mit den zuvor beschriebenen ersten Halteelementen 21 der Haltevorrichtung 20 zu ermöglichen. Die zweiten Halteelemente 22 sind dabei im Bereich des proximalen Endes 8 des Kanülenträgers 6 angeordnet. Im vorliegenden Ausführungsbeispiel sind im Halteansatz 15 die die zweiten Halteelemente 22 bildenden Ausnehmungen 25 angeordnet oder ausgebildet.

Durch diese radiale Verstellung bzw. Verlagerung der jeweils an einem Haltearm 24 angeordneten Vorsprünge 23 kommen diese außer Eingriff mit den Ausnehmungen 25, wodurch die Haltevorrichtung 20 außer Eingriff kommt. Sobald der Eingriff der Haltevorrichtung 20 gelöst ist, kommt das Stellelement 16 zur Wirkung und verlagert selbsttätig und automatisch den Kanülenträger 6 und somit auch die Kanüle 10 in deren abgedeckte Stellung (zweite Stellung oder Schutzstellung).

Um eine neuerliche Verwendung der Kanülenanordnung 1 zu vermeiden, kann der Kanülenträger 6 in seiner zweiten relativen Stellung bezüglich des Basiskörpers 2 mittels einer im Bereich des proximalen Endes 4 des Basiskörpers 2 angeordneten Arretiervorrichtung 26 in Axialrichtung relativ bezüglich des Basiskörpers arretiert an diesem gehaltert sein. Diese Arretiervorrichtung 26 kann derart ausgebildet sein, dass in der zweiten Stellung des Kanülenträgers 6 dieser in Axialrichtung gesehen, in beide Richtungen an einer erneuten Verstellung gehindert ist. Damit kann verhindert werden, dass der Kanülenträger 6 sich vom Basiskörper 2 löst und gleichzeitig auch eine erneute Rückstellung des Kanülenträgers 6 in die erste Stellung verhindert ist. Dazu sind mehrere erste Arretierelemente 27 und bevorzugt mehrere zweite Arretierelemente 28 vorgesehen. Die ersten Arretierelemente 27 sind im Bereich des proximalen Endes 4 des Basiskörpers 2 und die zweiten Arretierelemente 28 sind im Bereich des distalen Endes 7 des Kanülenträgers 6 angeordnet.

Weiters kann am Basiskörper 2 eine von diesem in Radialrichtung abstehende Flügelanordnung 29 angeordnet oder ausgebildet sein. Die Flügel der Flügelanordnung 29 können in einer aneinander anliegenden Stellung während des Einstichvorgangs zur besseren Halterung durch die Bedienperson dienen. Ist die Kanülenanordnung 1 mit ihrer Kanüle 10 für die Entnahme von Flüssigkeit aus dem Körper oder zum Zuführen von Flüssigkeiten in den Körper eingestochen, kann die gesamte Kanülenanordnung 1 mit den Flügeln der Flügelanordnung 29 mit einem zusätzlichen Klebestreifen oder einem anderen Haltemittel an der nicht näher dargestellten Oberfläche des Körpers gehalten bzw. fixiert werden.

Die Kanülenanordnung 1 ist in ihrem ungebrauchten Zustand zumeist in einer sterilen Verpackung abgepackt und wird aus dieser durch eine Bedienperson aus dieser entnommen. Dabei befindet sich der Kanülenträger 6 mitsamt der daran gehaltenen Kanüle 10 in der ersten Stellung, bei welcher die Kanüle 10 das distale Ende 3 des Basiskörpers 2 überragt. Um in diesem Zustand eine Stichverletzung zu vermeiden, ist in der Fig. 1 in strichlierten Linien eine zusätzliche, bevorzugt rohrförmig ausgebildete, Schutzhülle 30 angedeutet. Die Schutzhülle 30 kann nur durch einen rohrförmigen, an beiden Enden offenen Bauteil gebildet sein, wobei dann die gesamte Sicherheitsnadelanordnung 1 in einer zusätzlichen Überverpackung, z.B. einer nicht dargestellten Blisterhülle, bis zur bestimmungsgemäßen Verwendung steril verpackt ist. Nach Entfernung der Schutzhülle 30 oder der Schutzkappe vom Basiskörper 2 liegt die Kanüle 10 frei und kann an der dafür vorgesehenen Einstichstelle in den Körper eingestochen werden.

Weiters ist noch angedeutet, dass am proximalen Ende des Halteansatzes 15 ein Schlauch 31 zur Verbindung mit einer weiteren medizinischen Komponente angeordnet sein kann.

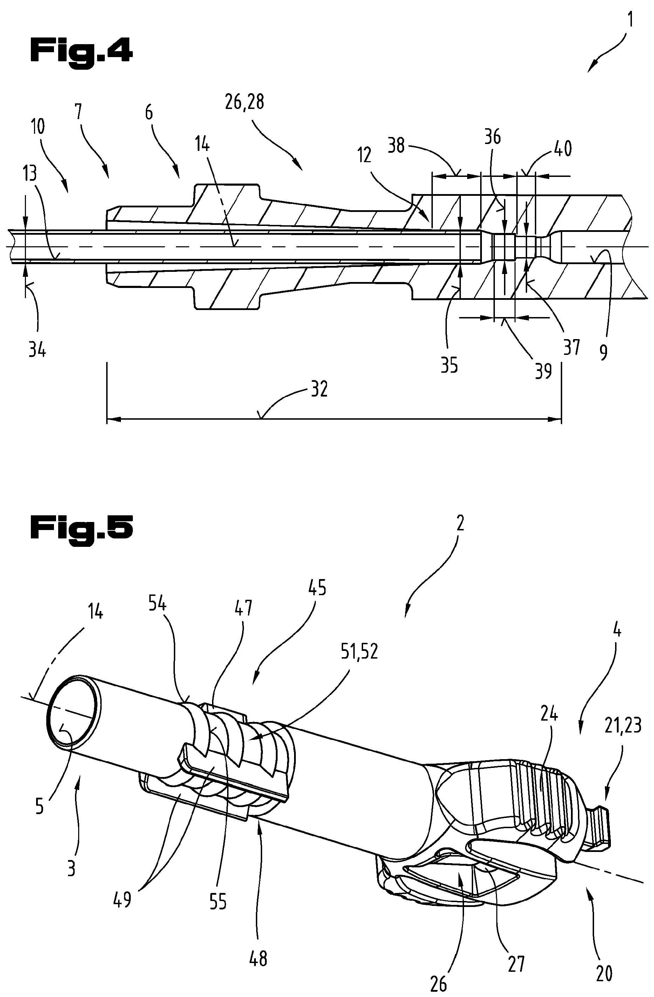

In der Fig. 4 ist der Kanülenträger 6 mit der daran befestigten Kanüle 10 im Axialschnitt gezeigt, um die Halterung der Kanüle 10 innerhalb des Durchströmkanals 9 im Kanülenträger 6 besser ersichtlich zu machen.

Der Durchströmkanal 9 definiert seinerseits weiters einen distalen Durchströmkanal-Endabschnitt 32 und einen proximalen Durchströmkanal-Endabschnitt 33. Die Kanüle 10 ist im distalen Durchströmkanal-Endabschnitt 32 aufgenommen und gehalten. Dies insbesondere mittels einer Klebeverbindung.

Je nach Verwendungszweck bzw. durchzuführender medizinischer Erfordernisse, sind Kanülen 10 mit zueinander unterschiedlichen Kanülen-Querschnittsabmessung 34 erforderlich. Diese können z.B. unterschiedliche Außendurchmesser aufweisen, wobei beispielhaft einige Außenabmessungen nachfolgend beispielhaft einige angeführt sind: 0,5 mm / 0,6 mm / 0,7 mm / 0,8 mm / 0,9 mm.

Bei diesem Kanülenträger 6 ist nun vorgesehen, dass im Axialschnitt gesehen der distale Durchströmkanal-Endabschnitt 32 ausgehend vom distalen Ende 7 des Kanülenträgers 6 in Richtung auf das proximale Ende 8 des Kanülenträgers 6 eine abgestuft ausgebildete Querschnittsform mit einer ersten Querschnittsabmessung 35 und zumindest einer weiteren Querschnittsabmessung 36, 37 aufweist. Im vorliegenden Ausführungsbeispiel sind drei zueinander unterschiedliche Querschnittsabmessung 35, 36, 37 vorgesehen, wobei dies nur als Beispiel anzusehen ist. Es können auch noch mehr als drei zueinander unterschiedliche Querschnittsabmessungen oder auch weniger vorgesehen werden. Dies gilt auch für die nachfolgend beschriebenen Aufnahmeabschnitte 38, 39, 40.

Jede der einzelnen Querschnittsabmessungen 35, 36, 37 definiert ihrerseits einen eigenen Aufnahmeabschnitt 38, 39, 40. Diese können analog zu den Querschnittsabmessungen jeweils als erster, zweiter und dritter Aufnahmeabschnitt bezeichnet werden. In jedem der unterschiedlichen Aufnahmeabschnitte 38, 39, 40 kann die entsprechende Kanüle 10 mit dem dazu korrespondierenden Kanülen-Querschnittsabmessung 34 aufgenommen werden.

Die Querschnittsabmessung 35, 36, 37 jedes Aufnahmeabschnitts 38, 39, 40 ist ausgehend vom distalen Ende 7 des Kanülenträgers 6 in Richtung auf das proximale Ende 8 des Kanülenträgers 6 jeweils geringer ist als die Querschnittsabmessung 37, 36, 35 des unmittelbar in Axialrichtung vorgeordneten Aufnahmeabschnitts 40, 39, 38. Der erste Aufnahmeabschnitt 38 ist dem zweiten Aufnahmeabschnitt 39 und dieser, falls vorhanden, ist dem dritten Aufnahmeabschnitt 40 vorgeordnet. Bevorzugt sind die Aufnahmeabschnitte 38, 39, 40 jeweils mit einem hohlzylinderförmigen Querschnitt ausgebildet.

Jede der zueinander unterschiedlichen Querschnittsabmessungen 35, 36, 37 der Aufnahmeabschnitte 38, 39, 40 ist derart ausgebildet, um jeweils jene Kanüle 10 mit der dazu korrespondierenden Kanülen-Querschnittsabmessung 34 aufzunehmen. Damit wird erreicht, dass in jeder der zueinander unterschiedlichen Querschnittsabmessungen 35, 36, 37 der Aufnahmeabschnitte 38, 39, 40 nur die jeweilige dazu korrespondierende Kanüle 10 mit deren Kanülen-Querschnittsabmessung aufgenommen werden kann, da die jeweilige Querschnittsabmessung 35, 36, 37 des jeweiligen Aufnahmeabschnitts 38, 39, 40 an die jeweils darin aufzunehmende und zu haltende Kanüle 10 mit deren Kanülen-Querschnittsabmessung abgestimmt und angepasst ist. Zumeist handelt es sich je nach späterem Einsatzzweck um vorbestimmte Kanülen-Querschnittsabmessungen, wobei die einzelnen zueinander unterschiedlichen Querschnittsabmessungen 35, 36, 37 der Aufnahmeabschnitte 38, 39, 40 so gewählt sind, dass die jeweilige Kanüle 10 in den dafür vorgesehenen der Aufnahmeabschnitte 38, 39, 40 eingesetzt und gegebenenfalls auch noch mittels eines zusätzlichen Haft- oder Klebemittels darin gehalten und befestigt wird oder ist. Der Einfachheit halber wurde nur ein Bezugszeichen für die Kanüle 10 mit den jeweils zueinander unterschiedlichen Kanülen-Querschnittsabmessungen 34 verwendet, da auch nur eine einzige Abmessung dargestellt ist. Im vorliegenden Ausführungsbeispiel ist jene Kanüle 10 mit der zur ersten Querschnittsabmessung 35 des ersten Aufnahmeabschnitts 38 korrespondierenden Kanülen-Querschnittsabmessungen 34 dargestellt.

Jeder der Aufnahmeabschnitte 38, 39, 40 bildet an seinem proximalen Ende jeweils einen Axialanschlag für das proximale Ende 12 der jeweiligen darin aufgenommen Kanüle 10. Eine in axialer Richtung vorbestimmte relative Positionierung der Kanüle 10 im jeweiligen Aufnahmeabschnitt 38, 39, 40 wird dann erzielt, wenn das jeweilige proximale Ende 12 der Kanüle 10 an jenem dazu korrespondierenden Axialanschlag des jeweiligen Aufnahmeabschnitts 38 oder 39 oder 40 daran in Richtung auf das proximale Ende 8 des Kanülenträgers 6 anliegend abgestützt ist.

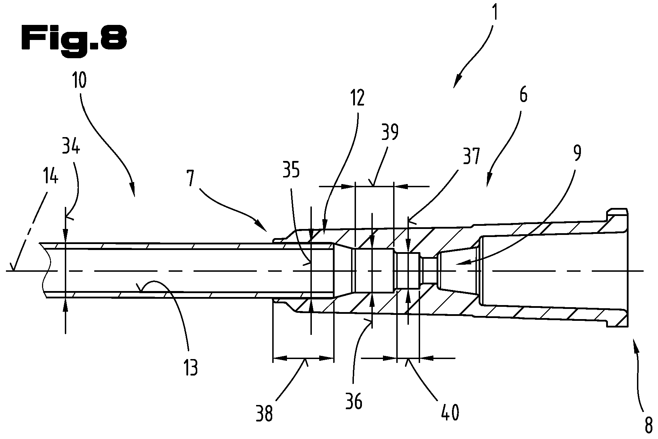

Im vorliegenden Ausführungsbeispiel ist der die Kanüle 10 tragende bzw. haltende Kanülenträger 6 eine hohlzylinderförmige Bauteilkomponente einer Sicherheits-Kanülenanordnung. Es wäre aber unabhängig davon möglich, die Kanüle 10 an einem als weiblichen Luer-Kopplungsteil ausgebildeten Kanülenträger 6 zu halten, wie dies vereinfacht in der nachfolgenden Fig. 8 dargestellt worden ist.

Wie zuvor beschrieben, dient die Arretiervorrichtung 26 mit den zusammenwirkenden ersten und zweiten Arretierelementen 27, 28 dazu, bei sich in der zweiten Stellung befindlichem Kanülenträger 6 diesen in seiner relativen Lage bezüglich des Basiskörper 2 unverschieblich positioniert zu arretieren. Damit soll eine Wiederverwendung und/oder auch ungewollte Stichverletzungen vermieden werden.

Die ersten Arretierelemente 27 der Arretiervorrichtung 26 sind als einseitig gelagerte Arretierarme ausgebildet, welche in bekannter Weise mit dem als über den Umfang durchlaufend ausgebildete Kreisringfläche (entspricht den zweiten Arretierelementen 28) zusammenwirkt. Um eine Materialermüdung und den damit einhergehenden Abbau der Rückstellkraft in einer vorgespannten Stellung der ersten Arretierelemente 27 zu vermeiden, ist zumindest eine Entlastungsvertiefung 41 im Kanülenträger 6 angeordnet oder ausgebildet. Diese ist am Besten in der Fig. 3 ersichtlich. Damit ist es möglich, dass bei sich in der ersten Stellung befindlichem Kanülenträger 6 die ersten Arretierelemente 27 in ihrer unverformten Stellung in die zumindest eine Entlastungsvertiefung 41 hineinragen. Die Entlastungsvertiefung 41 kann z.B. durch eine bevorzugt umlaufend ausgebildete nutförmige Vertiefung oder aber durch einen kegelstumpfförmig oder keilförmig ausgebildeten Teilabschnitt des Kanülenträgers 6 realisiert sein.

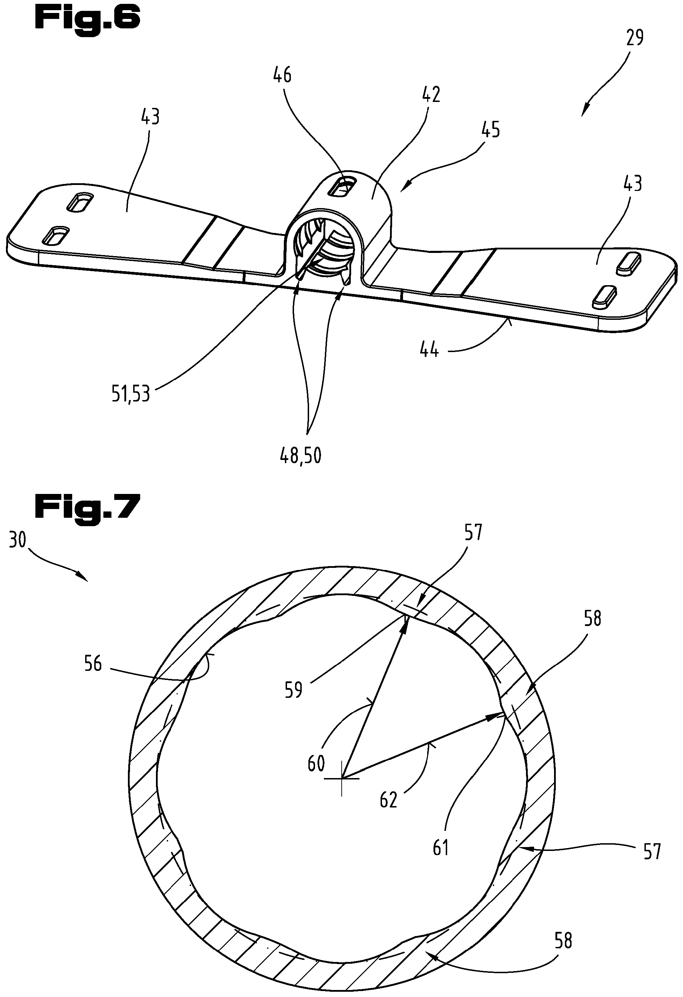

In den Fig. 5 und 6 ist die Flügelanordnung 29 und deren Halterung bzw. Befestigung am Basiskörper 2 detaillierter gezeigt und beschrieben. Die Flügelanordnung 29 umfasst ihrerseits einen überwiegend rohrförmig ausgebildeten Haltekörper 42 sowie jeweils vom Haltekörper 42 beidseits abstehende Flügel 43. Weiters ist noch eine ausschließlich auf mechanischer Basis beruhende Kopplungseinheit 45 zwischen dem Inneren des Haltekörpers 42 und dem Äußeren des Basiskörper 2 angeordnet oder ausgebildet. Mittels der Kopplungseinheit 45 ist die Flügelanordnung 29 in einer Kopplungsstellung am Basiskörper 2 gehalten.

Um einen Ansticherfolg optisch erkennbar zu machen, sind zumindest der Basiskörper 2 und auch der Kanülenträger 6 aus einem durchscheinenden oder einem durchsichtigen Werkstoff, zumeist einem Kunststoffwerkstoff, gebildet. Tritt das Blut aus dem proximalen Ende 12 der Kanüle 10 aus, kann dies optisch erkannt werden. Dazu ist hier weiters noch vorgesehen, dass im rohrförmig ausgebildeten Haltekörper 42 der Flügelanordnung 29 ein Sichtfenster 46 an einer von der Anlageseite 44 abgewendeten Seite angeordnet ist. Das Sichtfenster 46 ist voll umfänglich vom Material des Haltekörpers 42 umgeben und durchsetzt den Haltekörper 42 vollständig in radialer Richtung und somit in Richtung auf die Längsachse 14.

Aus einer Zusammenschau der Fig. 1, 2, 5 und 6 ist zu ersehen, dass am Basiskörper 2 ein in radialer Richtung abstehender Ansatz 47 angeordnet oder ausgebildet ist, welcher bei sich in der Kopplungsstellung befindlicher Flügelanordnung 29 am Basiskörper 2 in das Sichtfenster 46 hineinragt.

Zur Verhinderung einer relativen Verdrehung bzw. Verschwenkung der Flügelanordnung 29 bezüglich des Basiskörpers 2 um die Längsachse 14 zu erschweren oder gänzlich zu unterbinden, umfasst die Kopplungseinheit 45 weiters noch Verdreh-Sicherungsvorrichtung 48 mit zumindest einem ersten Verdreh-Sicherungselement 49 am oder im Basiskörper 2 und zumindest ein damit zusammenwirkendes zweites Verdreh-Sicherungselement 50 im oder am Haltekörper 42. Im vorliegenden Ausführungsbeispiel ist das erste Verdreh-Sicherungselement 49 oder sind die ersten Verdreh-Sicherungselemente 49 als Steg ausgebildet. Das zweite Verdreh-Sicherungselement 50 ist oder die zweiten Verdreh-Sicherungselemente 50 sind als Nut ausgebildet. Die Verdreh-Sicherungselemente 49, 50 weisen jeweils eine parallele Längsausrichtung bezüglich der Längsachse 14 auf. Die jeweils zusammenwirkenden Verdreh-Sicherungselemente 48, 50 sind im Radialschnitt gesehen und bei horizontaler Ausrichtung der Anlageseite 44, welche zumeist eine Ebene definiert, beidseits einer Vertikalebene und voneinander beabstandet angeordnet oder ausgebildet. Die jeweils zusammenwirkenden Verdreh-Sicherungselemente 48, 50 sind zueinander gegengleich ausgebildet.

Um auch eine in entgegengesetzter Richtung zu der in proximaler Richtung erfolgenden Aufschubbewegung ausgerichtete Trennbewegung der Flügelanordnung 29 vom Basiskörper 2 zu verhindern, ist eine Axial-Sicherungsvorrichtung 51 mit zumindest einem ersten Axial-Sicherungselement 52 am oder im Basiskörper 2 und zumindest ein damit zusammenwirkendes zweites Axial-Sicherungselement 53 im oder am Haltekörper 42 vorgesehen. Die Axial-Sicherungsvorrichtung 51 kann als Bestandteil der Kopplungseinheit 45 angesehen werden.

Der Basiskörper 2 bildet für das proximale Ende des Haltekörpers 42 einen in axialer Richtung wirkenden Anschlag aus, welcher durch eine Verdickung des Basiskörpers 2 ausgebildet sein kann. Im vorliegenden Ausführungsbeispiel sind die ersten Axial-Sicherungselemente 52 im Axialschnitt gesehen in etwa mit einem Dreieckquerschnitt ausgebildet. Eine erste Begrenzungslinie 54 des Dreieckquerschnitts definiert in axialer Aufschubrichtung der Flügelanordnung 29 auf den Basiskörper 2 gesehen eine auf die von der Längsachse 14 abgewendete Seite ansteigend ausgebildete Rampe. Eine zweite Begrenzungslinie 55 des Dreieckquerschnitts ist bevorzugt in einer Normalebene bezüglich der Längsachse 14 verlaufend ausgerichtet. Die zweiten Axial-Sicherungselemente 53 sind gegengleich bezüglich der ersten Axial-Sicherungselemente 52 auszubilden. Bevorzugt sind die ersten und zweiten Axial-Sicherungselemente 52, 53 über den Umfang durchlaufend ausgebildet bzw. angeordnet und können von den zuvor beschriebenen Verdreh-Sicherungselementen 49, 50 unterbrochen sein.

In der Fig. 7 ist ein möglicher Querschnitt der überwiegend rohrförmig oder hohlzylinderförmig ausgebildeten Schutzhülle 30 gezeigt, welche auch als Schutzhülse bezeichnet werden kann. Die Schutzhülle 30 dient dazu, am distalen Ende 3 des Basiskörpers 2 angeordnet und an diesem abnehmbar gehalten zu werden. Bevorzugt wird die Schutzhülle 30 aus einem Kunststoffmaterial in einem Extrusionsvorgang hergestellt und vom ansonsten endlosen Rohrkörper werden die einzelnen Schutzhüllen 30 mit der vorbestimmten Baulänge davon abgetrennt. Grundsätzlich wird eine umlaufende Hüllenwand gebildet, welche eine ausreichende Eigensteifigkeit aufweist. Die Hüllenwand ohne der nachfolgend beschriebenen Längsrippen 57, 58 kann z.B. eine Wandstärke in einem Bereich mit einer unteren Grenze von 0,25 mm und einer oberen Grenze von 0,35 mm, bevorzugt von 0,3 mm aufweisen. Die ideale, unverformte Wandstärke der Hüllenwand ist innenseitig mit einer strich-punktierten Linie angedeutet.

In der ersten Stellung des Kanülenträgers 6 wird von der Schutzhülle 30 die vom Basiskörper 2 in distaler Richtung vorragende Kanüle 10 abgedeckt. Um einerseits eine ausreichende Halte- bzw. Haftkraft der Schutzhülle 30 am Basiskörper 2 und andererseits eine innerhalb gewisser Grenzen definierte Abzugskraft der Schutzhülle 30 vom Basiskörper 2 zu erzielen, sind an einer Innenfläche 56 der Hüllenwand der Schutzhülle 30 mehrere über den Umfang verteilt angeordnete erste Längsrippen 57 und zweite Längsrippen 58 ausgebildet oder vorgesehen. Es sind jeweils zumindest zwei erste Längsrippen 57 und zumindest zwei zweite Längsrippen 58 vorgesehen. Die über den Umfang verteilt angeordneten Längsrippen 57 und 58 sind in Umfangsrichtung jeweils abwechseln zueinander angeordnet, also eine erste Längsrippe 57 und nachfolgend eine zweite Längsrippe 58 usw..

Bei unverformtem Querschnitt der Schutzhülle 30 enden erste Enden 59 der ersten Längsrippen 57 jeweils in einer ersten Radialdistanz 60 vor der Längsachse 14. Es enden ebenfalls bei einem unverformten Querschnitt der Schutzhülle 30 zweite Enden 61 der zweiten Längsrippen 58 in einer zweiten Radialdistanz 62 vor der Längsachse 14. Die erste Radialdistanz 60 ist größer ausgebildet als die zweite Radialdistanz 62, wobei die zweiten Enden 61 der zweiten Längsrippen 58 der Längsachse 14 näher liegen. Da jeweils die ersten Radialdistanzen 60 und jeweils die zweiten Radialdistanz 62 zueinander gleich groß ausgebildet sind, liegen die ersten Enden 59 der ersten Längsrippen 57 jeweils auf einer ersten Kreisbahn und die zweiten Enden 61 der zweiten Längsrippen 58 jeweils auf einer zweiten Kreisbahn. Die beiden Kreisbahnen sind konzentrisch bezüglich der Längsachse 14 angeordnet. Ein Differenzwert der erste Radialdistanz 60 abzüglich der zweiten Radialdistanz 62 ist aus einem Differenz-Wertebereich gewählt, dessen untere Grenze 0,01 mm, bevorzugt 0,04 mm, und dessen obere Grenze 0,1 mm, bevorzugt 0,06 mm, beträgt. Ein bevorzugter Differenzwert kann z.B. auch 0,05 mm betragen. Die Gesamtanzahl der ersten Längsrippen 57 und der zweiten Längsrippen 58 ist eine gerade Anzahl und ist aus einer Gesamtanzahl von vier, sechs, acht oder zehn ausgewählt. Bevorzugt können die ersten Längsrippen 57 und/oder die zweiten Längsrippen 58 im Radialschnitt gesehen jeweils eine Wellenform und/oder wellenförmig ausgebildete Übergange aufweisen.

Aufgrund der zueinander unterschiedlich gewählten Radialdistanzen 60 und 62 und die abwechselnde Anordnung über den Umfang gesehen, können Fertigungstoleranzen bei der Schutzhülle 30 und dem Basiskörper 2 besser zueinander ausgeglichen werden und trotzdem die zuvor beschriebene Halte- bzw. Haftkraft und die erforderliche Abzugskraft in vorbestimmten Grenzen realisiert bzw. eingehalten werden. Bevorzugte Abzugskräfte liegen in einem Kraft-Wertebereich mit einer unteren Grenze von 0,5N, bevorzugt 1N, und einer Oberen Grenze von 10N, bevorzugt 4N.

Im Montagezustand der Schutzhülle 30 am dafür vorgesehenen Ansatz des Basiskörpers 2, welcher Ansatz eine zylindrische Außenfläche aufweist, liegen die zweiten Längsrippen 58 aufgrund der geringeren zweiten Radialdistanz 62 an der Außenfläche an. Je nach den erzielten Fertigungstoleranzen können die ersten Längsrippen 57 mit ihren ersten Enden 59 bedingt durch die elastischen Eigenschaften des Kunststoffwerkstoffs der Schutzhülle 30 und der größeren ersten Radialdistanz 60 ebenfalls mehr oder weniger zur Anlage an der Außenfläche des Ansatzes des Basiskörpers 2 gebracht werden. Die Umfangswand der Schutzhülle 30 wird im Bereich der ersten Längsrippen 57 von ihrer idealen Kreisform zu einem Bogenabschnitt mit einem zur idealen Kreisform dazu größeren Bogenradius umgeformt. Damit kann in diesen Bogenabschnitten der ersten Längsrippen 57 die ansonsten aufgebaute Radialkraft in Richtung auf die Längsachse 14 in gewissen Grenzen reduziert werden. Sind z.B. vier erste Längsrippen 57 und dazwischen die vier zweiten Längsrippen 58 kommt eine annähernde 4-Eck Ausrichtung im Montagezustand zustande.

Weiters kann noch vorgesehen sein, dass zur leichteren Gleitbewegung des Kanülenträgers 6 innerhalb der Durchgangsöffnung 5 des Basiskörper 2 auf zumindest einer Oberfläche der zuvor aufgezählten Bauteile eine Beschichtung aufgebracht ist. In vorteilhafter Weise kann die gleiche Beschichtung eingesetzt werden, mit welcher auch die Kanüle 10 beschichtet wird. Bei einer derartigen Beschichtung kann es sich z.B. um eine Silikonbeschichtung handeln, welche nach deren Aufbringen auf die Substratoberfläche aushärtend ausgebildet ist. Das im Beschichtungsmittel enthaltene Lösungsmittel verdunstet nach dem Beschichtungsvorgang und das verbleibende Beschichtungsmittel verbindet sich mit dem Trägersubstrat, auf welchem es aufgebracht worden ist.

Die Ausführungsbeispiele zeigen mögliche Ausführungsvarianten, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

**Bezugszeichenaufstellung**

| | | | |
|---|---|---|---|
| 1 | Kanülenanordnung | 31 | Schlauch |
| 2 | Basiskörper | 32 | distaler Durchströmkanal-Endab- |
| 3 | distales Ende | | schnitt |
| 4 | proximales Ende | 33 | proximaler Durchströmkanal-End-abschnitt |
| 5 | Durchgangsöffnung | | |
| 6 | Kanülenträger | 34 | Kanülen-Querschnittsabmessung |
| 7 | distales Ende | 35 | erste Querschnittsabmessung |
| 8 | proximales Ende | 36 | zweite Querschnittsabmessung |
| 9 | Durchströmkanal | 37 | dritte Querschnittsabmessung |
| 10 | Kanüle | 38 | erster Aufnahmeabschnitt |
| 11 | distales Ende | 39 | zweiter Aufnahmeabschnitt |
| 12 | proximales Ende | 40 | dritter Aufnahmeabschnitt |
| 13 | Strömungskanal | 41 | Entlastungsvertiefung |
| 14 | Längsachse | 42 | Haltekörper |
| 15 | Halteansatz | 43 | Flügel |
| 16 | Stellelement | 44 | Anlageseite |
| 17 | distales Stellelementende | 45 | Kopplungseinheit |
| 18 | proximales Stellelementende | 46 | Sichtfenster |
| 19 | Aufnahmeöffnung | 47 | Ansatz |
| 20 | Haltevorrichtung | 48 | Verdreh-Sicherungsvorrichtung |
| 21 | erstes Halteelement | 49 | erstes Verdreh-Sicherungselement |
| 22 | zweites Halteelement | 50 | zweites Verdreh-Sicherungselement |
| 23 | Vorsprung | | |
| 24 | Haltearm | 51 | Axial-Sicherungsvorrichtung |
| 25 | Ausnehmung | 52 | erstes Axial-Sicherungselement |
| 26 | Arretiervorrichtung | 53 | zweites Axial-Sicherungselement |
| 27 | erstes Arretierelement | 54 | erste Begrenzungslinie |
| 28 | zweites Arretierelement | 55 | zweite Begrenzungslinie |
| 29 | Flügelanordnung | 56 | Innenfläche |
| 30 | Schutzhülle | 57 | erste Längsrippe |
| 58 | zweite Längsrippe | | |
| 59 | erstes Ende | | |
| 60 | erste Radialdistanz | | |
| 61 | zweites Ende | | |
| 62 | zweite Radialdistanz | | |

## Patentansprüche

1. Kanülenanordnung (1), insbesondere Sicherheits-Kanülenanordnung, umfassend
- einen Kanülenträger (6) mit einem distalen Ende (7) und einem proximalen Ende (8), wobei sich innerhalb des Kanülenträgers (6) zwischen seinem distalen Ende (7) und seinem proximalen Ende (8) ein Durchströmkanal (9) erstreckt, und der Durchströmkanal (9) einen distalen Durchströmkanal-Endabschnitt (32) und einen proximalen Durchströmkanal-Endabschnitt (33) definiert,
- eine Kanüle (10) mit einem distalen Ende (11) und einem proximalen Ende (12), wobei sich innerhalb der Kanüle (10) zwischen ihrem distalen Ende (11) und ihrem proximalen Ende (12) ein Strömungskanal (13) erstreckt, und
- wobei das proximale Ende (12) der Kanüle (10) im distalen Durchströmkanal-Endabschnitt (32) des Kanülenträgers (6) aufgenommen und am Kanülenträger (6) gehalten ist, insbesondere mit diesem verklebt ist, und die Kanüle (10) und der Kanülenträger (6) eine Längsachse (14) definieren,
- wobei im Axialschnitt gesehen der distale Durchströmkanal-Endabschnitt (32) ausgehend vom distalen Ende (7) des Kanülenträgers (6) in Richtung auf das proximale Ende (8) des Kanülenträgers (6) eine abgestuft ausgebildete Querschnittsform mit einer ersten Querschnittsabmessung (35) und zumindest einer weiteren Querschnittsabmessung (36, 37) aufwei st,
- wobei jede der Querschnittsabmessungen (35, 36, 37) einen eigenen Aufnahmeabschnitt (38, 39, 40) für die jeweilige darin aufzunehmende Kanüle (10) definiert,
- dass die Querschnittsabmessung (35, 36, 37) jedes Aufnahmeabschnitts (38, 39, 40) ausgehend vom distalen Ende (7) des Kanülenträgers (6) in Richtung auf das proximale Ende (8) des Kanülenträgers (6) jeweils geringer ist als die Querschnittsabmessung (37, 36, 35) des unmittelbar in Axialrichtung vorgeordneten Aufnahmeabschnitts (40, 39, 38), und
- wobei jede der zueinander unterschiedlichen Querschnittsabmessungen (35, 36, 37) der Aufnahmeabschnitte (38, 39, 40) derart ausgebildet ist, um jeweils jene Kanüle (10) mit einer dazu korrespondierenden Kanülen-Querschnittsabmessung (34) aufnehmen zu können,
- wobei weiters ein Basiskörper (2) mit einem distalen Ende (3) und einem proximalen Ende (4) vorgesehen ist, wobei sich zwischen dem distalen Ende (3) des Basiskörpers (2) und dem proximalen Ende (4) des Basiskörpers (2) in diesem eine Durchgangsöffnung (5) erstreckt und dass zumindest der Kanülenträger (6) mit der daran gehaltenen Kanüle (10) in der Durchgangsöffnung (5) aufgenommen sowie in der Durchgangsöffnung (5) in Axialrichtung verstellbar ist, und
- wobei weiters eine Flügelanordnung (29) mit einem rohrförmig ausgebildeten Haltekörper (42) und vom Haltekörper (42) beidseits abstehenden Flügel (43) vorgesehen ist, wobei die Flügel (43) eine einem Patienten zuwendbare Anlageseite (44) definieren und dass weiters eine Kopplungseinheit (45) zwischen dem Haltekörper (42) und dem Basiskörper (2) angeordnet oder ausgebildet ist und mittels der Kopplungseinheit (45) die Flügelanordnung (29) in einer Kopplungsstellung am Basiskörper (2) gehalten ist,
**dadurch gekennzeichnet,**
- **dass** ein Sichtfenster (46) im rohrförmig ausgebildeten Haltekörper (42) ausgebildet ist, welches Sichtfenster (46) an einer von der Anlageseite (44) abgewendeten Seite angeordnet ist und dass in der Kopplungsstellung ein vom Basiskörper (2) in radialer Richtung abstehender Ansatz (47) in das Sichtfenster (46) hineinragt,
- wobei der Basiskörper (2) und der Kanülenträger (6) aus einem durchscheinenden oder einem durchsichtigen Werkstoff, zumeist einem Kunststoffwerkstoff, gebildet sind.

2. Kanülenanordnung (1) nach Anspruch 1, **dadurch gekennzeichnet, dass** die Aufnahmeabschnitte (38, 39, 40) jeweils mit einem hohlzylinderförmigen Querschnitt ausgebildet sind.

3. Kanülenanordnung (1) nach Anspruch 1 oder 2, **dadurch gekennzeichnet, dass** mehrere, insbesondere drei, in Axialrichtung hintereinander ausgebildete Aufnahmeabschnitte (38, 39, 40) vorgesehen sind.

4. Kanülenanordnung (1) nach einem der vorhergehenden Ansprüche, **dadurch gekennzeichnet, dass** jeder der Aufnahmeabschnitte (38, 39, 40) an seinem proximalen Ende jeweils einen Axialanschlag für das proximale Ende (12) der jeweiligen darin aufgenommen Kanüle (10) bildet.

5. Kanülenanordnung (1) nach Anspruch 4, **dadurch gekennzeichnet, dass** das jeweilige proximale Ende (12) der Kanüle (10) an jenem dazu korrespondierenden Axialanschlag des jeweiligen Aufnahmeabschnitts (38, 39, 40) daran in Richtung auf das proximale Ende (8) des Kanülenträgers (6) anliegend abgestützt ist.

6. Kanülenanordnung (1) nach einem der vorhergehenden Ansprüche, **dadurch gekennzeichnet, dass** der Kanülenträger (6) von einem weiblichen Luer-Kopplungsteil oder einer Bauteilkomponente einer Sicherheits-Kanülenanordnung gebildet ist.

7. Kanülenanordnung (1) nach einem der vorhergehenden Ansprüche, **dadurch gekennzeichnet, dass** weiters ein selbsttätig wirkendes Stellelement (16) vorgesehen ist, welches Stellelement (16) zwischen dem Basiskörper (2) und dem Kanülenträger (6) wirkend angeordnet ist, und das Stellelement (16) den Kanülenträger (6) mitsamt der daran gehaltenen Kanüle (10) von einer ersten Stellung, bei welcher ersten Stellung die Kanüle (10) über das distale Ende (3) des Basiskörpers (2) vorragt, in eine zweite Stellung verlagert, bei welcher zweiten Stellung zumindest das distale Ende (11) der Kanüle (10) vom Basiskörper (2) abgedeckt ist.

8. Kanülenanordnung (1) nach einem der vorhergehenden Ansprüche, **dadurch gekennzeichnet, dass** weiters eine Haltevorrichtung (20) mit mehreren ersten Halteelementen (21) und mehreren zweiten Halteelementen (22) vorgesehen ist, welche Haltevorrichtung (20) bei der sich in gegenseitigem Eingriff befindlichen Position der ersten und zweiten Halteelemente (21, 22) die erste Stellung des Kanülenträgers (6) bezüglich des Basiskörpers (2) festlegt, und dass die ersten Halteelemente (21) im Bereich des proximalen Endes (4) des Basiskörpers (2) und die zweiten Halteelemente (22) im Bereich des proximalen Endes (8) des Kanülenträgers (6) angeordnet sind.

9. Kanülenanordnung (1) nach einem der vorhergehenden Ansprüche, **dadurch gekennzeichnet, dass** weiters eine Arretiervorrichtung (26) mit mehreren ersten und zweiten Arretierelementen (27, 28) vorgesehen ist, welche Arretiervorrichtung (26) bei der sich in gegenseitigem Eingriff befindlichen Position der ersten und zweiten Arretierelemente (27, 28) die zweite Stellung des Kanülenträgers (6) bezüglich des Basiskörpers (2) festlegt, und dass die ersten Arretierelemente (27) im Bereich des proximalen Endes (4) des Basiskörpers (2) und die zweiten Arretierelemente (28) im Bereich des distalen Endes (7) des Kanülenträgers (6) angeordnet sind.

10. Kanülenanordnung (1) nach einem der vorhergehenden Ansprüche, **dadurch gekennzeichnet, dass** zumindest eine Entlastungsvertiefung (41) im Kanülenträger (6) angeordnet oder ausgebildet ist, und dass bei sich in der ersten Stellung befindlichem Kanülenträger (6) die ersten Arretierelemente (27) in ihrer unverformten Stellung in die zumindest eine Entlastungsvertiefung (41) hineinragen.

11. Kanülenanordnung (1) nach einem der vorhergehenden Ansprüche, **dadurch gekennzeichnet, dass** die Kopplungseinheit (45) weiters eine Verdreh-Sicherungsvorrichtung (48) mit zumindest einem ersten Verdreh-Sicherungselement (49) am oder im Basiskörper (2) und zumindest ein damit zusammenwirkendes zweites Verdreh-Sicherungselement (50) im oder am Haltekörper (42) umfasst.

12. Kanülenanordnung (1) nach Anspruch 11, **dadurch gekennzeichnet, dass** das zumindest eine erste Verdreh-Sicherungselement (49) als Steg und das zumindest eine zweite Verdreh-Sicherungselement (50) als Nut ausgebildet ist und dass die Verdreh-Sicherungselemente (49, 50) eine parallele Längsausrichtung bezüglich der Längsachse (14) aufweisen.

13. Kanülenanordnung (1) nach einem der vorhergehenden Ansprüche, **dadurch gekennzeichnet, dass** die Kopplungseinheit (45) weiters eine Axial-Sicherungsvorrichtung (51) mit zumindest einem ersten Axial-Sicherungselement (52) am oder im Basiskörper (2) und zumindest ein damit zusammenwirkendes zweites Axial-Sicherungselement (53) im oder am Haltekörper (42) umfasst.

14. Kanülenanordnung (1) nach Anspruch 13, **dadurch gekennzeichnet, dass** die Axial-Sicherungselemente (52, 53) im Axialschnitt gesehen in etwa mit einem Dreieckquerschnitt ausgebildet sind, wobei eine erste Begrenzungslinie (54) des Dreieckquerschnitts in axialer Aufschubrichtung der Flügelanordnung (29) auf den Basiskörper (2) gesehen eine auf die von der Längsachse (14) abgewendete Seite ansteigend ausgebildete Rampe definiert und eine zweite Begrenzungslinie (55) des Dreieckquerschnitts in einer Normalebene bezüglich der Längsachse (14) verlaufend ausgerichtet ist.

15. Kanülenanordnung (1) nach einem der vorhergehenden Ansprüche, **dadurch gekennzeichnet, dass** weiters eine insbesondere rohrförmig ausgebildete Schutzhülle (30) vorgesehen ist und die Schutzhülle (30) am distalen Ende (3) des Basiskörpers (2) angeordnet und an diesem abnehmbar gehalten ist, wobei von der Schutzhülle (30) die in der ersten Stellung vom Basiskörper (2) in distaler Richtung vorragende Kanüle (10) abgedeckt ist.

16. Kanülenanordnung (1) nach Anspruch 15, **dadurch gekennzeichnet, dass** zumindest zwei erste Längsrippen (57) und zumindest zwei zweite Längsrippen (58) an einer der Längsachse (14) zugewendeten Innenfläche (56) der Schutzhülle (30) über den Umfang verteilt angeordnet oder ausgebildet sind, und dass in Umfangsrichtung jeweils abwechseln eine erste Längsrippe (57) und eine zweite Längsrippe (58) angeordnet sind.

17. Kanülenanordnung (1) nach Anspruch 16, **dadurch gekennzeichnet, dass** bei unverformtem Querschnitt der Schutzhülle (30) erste Enden (59) der ersten Längsrippen (57) in einer ersten Radialdistanz (60) vor der Längsachse (14) enden und zweite Enden (61) der zweiten Längsrippen (58) in einer zweiten Radialdistanz (62) vor der Längsachse (14) enden, wobei die erste Radialdistanz (60) größer ausgebildet ist als die zweite Radialdistanz (62).

18. Kanülenanordnung (1) nach Anspruch 17, **dadurch gekennzeichnet, dass** die ersten Enden (59) der ersten Längsrippen (57) auf einer ersten Kreisbahn und die zweiten Enden (61) der zweiten Längsrippen (58) auf einer zweiten Kreisbahn liegend angeordnet sind und die beiden Kreisbahnen konzentrisch bezüglich der Längsachse (14) angeordnet sind.

19. Kanülenanordnung (1) nach Anspruch 17 oder 18, **dadurch gekennzeichnet, dass** ein Differenzwert der erste Radialdistanz (60) abzüglich der zweiten Radialdistanz (62) aus einem Differenz-Wertebereich stammt, dessen untere 0,01 mm, bevorzugt 0,04 mm, und dessen obere Grenze 0,1 mm, bevorzugt 0,06 mm, beträgt.

20. Kanülenanordnung (1) nach einem der Ansprüche 16 bis 19, **dadurch gekennzeichnet, dass** die Gesamtanzahl der ersten Längsrippen (57) und der zweiten Längsrippen (58) eine gerade Anzahl ist und aus einer Gesamtanzahl von vier, sechs, acht oder zehn ausgewählt ist.

21. Kanülenanordnung (1) nach einem der Ansprüche 16 bis 20, **dadurch gekennzeichnet, dass** die ersten Längsrippen (57) und/oder zweiten Längsrippen (58) im Radialschnitt gesehen jeweils eine Wellenform aufweisen.

## Claims

1. A cannula arrangement (1), in particular a safety cannula arrangement, comprising
- a cannula support (6) with a distal end (7) and a proximal end (8), wherein a flow-through channel (9) extends within the cannula support (6) between its distal end (7) and its proximal end (8), and the flow-through channel (9) defines a distal flow-through channel end section (32) and a proximal flow-through channel end section (33),
- a cannula (10) with a distal end (11) and a proximal end (12), wherein a flow channel (13) extends within the cannula (10) between its distal end (11) and its proximal end (12), and
- wherein the proximal end (12) of the cannula (10) is received in the distal flow-through channel end section (32) of the cannula support (6) and is held on the cannula support (6), in particular glued therewith, and the cannula (10) and the cannula support (6) define a longitudinal axis (14),
- wherein, as seen in axial section, the distal flow-through channel end section (32), starting from the distal end (7) of the cannula support (6) in the direction towards the proximal end (8) of the cannula support (6), has a cross-sectional shape of stepped design with a first cross-sectional dimension (35) and at least one further cross-sectional dimension (36, 37),
- wherein each one of the cross-sectional dimensions (35, 36, 37) defines an own receiving section (38, 39, 40) for the respective cannula (10) to be received therein,
- wherein the cross-sectional dimension (35, 36, 37) of each receiving section (38, 39, 40), starting from the distal end (7) of the cannula support (6) in the direction towards the proximal end (8) of the cannula support (6), is in each case smaller than the cross-sectional dimension (37, 36, 35) of the receiving section (40, 39, 38) immediately upstream in the axial direction, and
- wherein each one of the cross-sectional dimensions (35, 36, 37) of the receiving sections (38, 39, 40), which differ from one another, is embodied to receive the cannula (10) with a corresponding cross-sectional cannula dimension (34) in each case,
- wherein furthermore, a base body (2) with a distal end (3) and a proximal end (4) is provided, wherein a hole (5) extends in the base body (2) between the distal end (3) of the base body (2) and the proximal end (4) of the base body (2), and that at least the cannula support (6) with the cannula (10) held thereon is received in the hole (5) and is adjustable in the axial direction in the hole (5), and
- wherein furthermore, a wing arrangement (29) with a tube-shaped retaining body (42) and wings (43) projecting from the retaining body (42) on both sides is provided, wherein the wings (43) define a contact side (44) which can be positioned to face a patient, and that, furthermore, a coupling unit (45) is arranged or formed between the retaining body (42) and the base body (2), and the wing arrangement (29) is held in a coupling position on the base body (2) by means of the coupling unit (45),
**characterized in**
- **that** an observation window (46) is formed in the tube-shaped retaining body (42), said observation window (46) being arranged on a side facing away from the contact side (44), and that in the coupling position, a protuberance (47) projecting from the base body (2) in the radial direction projects into the observation window (46),
- wherein the base body (2) and the cannula support (6) are made of a translucent or transparent material, usually a plastic material.

2. The cannula arrangement (1) according to claim 1, **characterized in that** the receiving sections (38, 39, 40) are embodied so as to each have a hollow-cylindrical cross-section.

3. The cannula arrangement (1) according to claim 1 or 2, **characterized in that** multiple, in particular three, receiving sections (38, 39, 40) formed behind one another in the axial direction are provided.

4. The cannula arrangement (1) according to one of the preceding claims, **characterized in that** each one of the receiving sections (38, 39, 40) on its proximal end forms an axial stop for the proximal end (12) of the respective cannula (10) received therein in each case.

5. The cannula arrangement (1) according to claim 4, **characterized in that** the respective proximal end (12) of the cannula (10) is supported on the corresponding axial stop of the respective receiving section (38, 39, 40) resting thereon the direction towards the proximal end (8) of the cannula support (6).

6. The cannula arrangement (1) according to one of the preceding claims, **characterized in that** the cannula support (6) is formed by a female Luer coupling part or a component part of a safety cannula arrangement.

7. The cannula arrangement (1) according to one of the preceding claims, **characterized in that**, furthermore, an automatically acting adjusting element (16) is provided, said adjusting element (16) being arranged acting between the base body (2) and the cannula support (6), and the adjusting element (16) displaces the cannula support (6) together with the cannula (10) held thereon from a first position, in which first position the cannula (10) projects beyond the distal end (3) of the base body (2), into a second position, in which second position at least the distal end (11) of the cannula (10) is covered by the base body (2).

8. The cannula arrangement (1) according to one of the preceding claims, **characterized in that**, furthermore, a retaining device (20) with multiple first retaining elements (21) and multiple second retaining elements (22) is provided, said retaining device (20) defining the first position of the cannula support (6) with respect to the base body (2) in the mutually engaged position of the first and second retaining elements (21, 22), and that the first retaining elements (21) are arranged in the region of the proximal end (4) of the base body (2) and the second retaining elements (22) are arranged in the region of the proximal end (8) of the cannula support (6).

9. The cannula arrangement (1) according to one of the preceding claims, **characterized in that**, furthermore, an arresting device (26) with multiple first and second arresting elements (27, 28) is provided, said arresting device (26) defining the second position of the cannula support (6) with respect to the base body (2) in the mutually engaged position of the first and second arresting elements (27, 28), and that the first arresting elements (27) are arranged in the region of the proximal end (4) of the base body (2) and the second arresting elements (28) are arranged in the region of the distal end (7) of the cannula support (6).

10. The cannula arrangement (1) according to one of the preceding claims, **characterized in that** at least one relief recess (41) is arranged or formed in the cannula support (6), and that, when the cannula support (6) is in the first position, the first arresting elements (27) project into the at least one relief recess (41) in their undeformed position.

11. The cannula arrangement (1) according to one of the preceding claims, **characterized in that** the coupling unit (45) further comprises an anti-rotation device (48) with at least one first anti-rotation element (49) on or in the base body (2) and at least one second anti-rotation element (50) cooperating therewith in or on the retaining body (42).

12. The cannula arrangement (1) according to claim 11, **characterized in that** the at least one first anti-rotation element (49) is embodied as a web and the at least one second anti-rotation element (50) is embodied as a groove, and that the anti-rotation elements (49, 50) have a parallel longitudinal alignment with respect to the longitudinal axis (14).

13. The cannula arrangement (1) according to one of the preceding claims, **characterized in that** the coupling unit (45) further comprises an axial securing device (51) with at least one first axial securing element (52) on or in the base body (2) and at least one second axial securing element (53) cooperating therewith in or on the retaining body (42).

14. The cannula arrangement (1) according to claim 13, **characterized in that** the axial securing elements (52, 53), as seen in axial section, are formed approximately with a triangular cross-section, wherein a first boundary line (54) of the triangular cross-section, as viewed in the axial push-on direction of the wing arrangement (29) onto the base body (2), defines a ramp formed to rise on the side facing away from the longitudinal axis (14), and a second boundary line (55) of the triangular cross-section is oriented to run in a normal plane with respect to the longitudinal axis (14).

15. The cannula arrangement (1) according to one of the preceding claims, **characterized in that**, furthermore, an in particular tube-shaped protective cover (30) is provided and the protective cover (30) is arranged on the distal end (3) of the base body (2) and is detachably held thereon, wherein the cannula (10) projecting from the base body (2) in distal direction in the first position is covered by the protective cover (30).

16. The cannula arrangement (1) according to claim 15, **characterized in that** at least two first longitudinal ribs (57) and at least two second longitudinal ribs (58) are arranged or formed distributed across the circumference on an internal surface (56) of the protective cover (30) facing the longitudinal axis (14), and that a first longitudinal rib (57) and a second longitudinal rib (58) are arranged alternately in the circumferential direction in each case.

17. The cannula arrangement (1) according to claim 16, **characterized in that**, in the undeformed cross-section of the protective cover (30), first ends (59) of the first longitudinal ribs (57) end at a first radial distance (60) in front of the longitudinal axis (14) and second ends (61) of the second longitudinal ribs (58) end at a second radial distance (62) in front of the longitudinal axis (14), wherein the first radial distance (60) is larger than the second radial distance (62).

18. The cannula arrangement (1) according to claim 17, **characterized in that** the first ends (59) of the first longitudinal ribs (57) are arranged on a first circular path and the second ends (61) of the second longitudinal ribs (58) are arranged on a second circular path and the two circular paths are arranged concentrically with respect to the longitudinal axis (14).

19. The cannula arrangement (1) according to claim 17 or 18, **characterized in that** a difference value of the first radial distance (60) minus the second radial distance (62) is in a difference value range the lower limit of which is 0.01 mm, preferably 0.04 mm, and the upper limit of which is 0.1 mm, preferably 0.06 mm.

20. The cannula arrangement (1) according to one of claims 16 to 19, **characterized in that** it is provided that the total number of the first longitudinal ribs (57) and the second longitudinal ribs (58) is an even number and is selected from a total number of four, six, eight or ten.

21. The cannula arrangement (1) according to one of claims 16 to 20, **characterized in that** the first longitudinal ribs (57) and/or second longitudinal ribs (58), as seen in radial section, each have a wave shape.

## Revendications

1. Ensemble de canules (1), en particulier ensemble de canules de sécurité, comprenant
- un support de canule (6) avec une extrémité distale (7) et une extrémité proximale (8), un canal de passage (9) s'étendant à l'intérieur du support de canule (6) entre son extrémité distale (7) et son extrémité proximale (8), et le canal de passage (9) étant défini par une section d'extrémité distale de canal de passage (32) et une section d'extrémité proximale de canal de passage (33),
- une canule (10) avec une extrémité distale (11) et une extrémité proximale (12), un canal d'écoulement (13) s'étendant à l'intérieur de la canule (10) entre son extrémité distale (11) et son extrémité proximale (12), et
- l'extrémité proximale (12) de la canule (10) étant logée dans la section d'extrémité distale du canal de passage (32) du support de canule (6) et maintenue sur le support de canule (6), en particulier collée à celui-ci, et la canule (10) et le support de canule (6) définissant un axe longitudinal (14),
- la section d'extrémité distale du canal de passage (32) présentant, vue en coupe axiale, à partir de l'extrémité distale (7) du support de canule (6) vers l'extrémité proximale (8) du support de canule (6), une forme de section transversale étagée avec une première dimension de section transversale (35) et au moins une autre dimension de section transversale (36, 37),
- chacune des dimensions de sections transversales (35, 36, 37) définissant une section de logement (38, 39, 40) propre pour la canule (10) respective à loger dans celle-ci,
- en ce que la dimension de section transversale (35, 36, 37) de chaque section de logement (38, 39, 40) partant de l'extrémité distale (7) du support de canule (6) dans la direction de l'extrémité proximale (8) du support de canule (6) est respectivement inférieure à la dimension de section transversale (37, 36, 35) de la section de logement (40, 39, 38) située immédiatement en amont dans la direction axiale, et
- chacune des dimensions de sections transversales (35, 36, 37) différentes les unes des autres des sections de logement (38, 39, 40) étant conçue de manière à pouvoir loger respectivement cette canule (10) avec une dimension de section transversale de canule (34) correspondante,
- un corps de base (2) avec une extrémité distale (3) et une extrémité proximale (4) étant en outre prévu, une ouverture de passage (5) s'étendant entre l'extrémité distale (3) du corps de base (2) et l'extrémité proximale (4) du corps de base (2) dans celui-ci, et au moins le support de canule (6) avec la canule (10) maintenue sur celui-ci étant logé dans l'ouverture de passage (5) et pouvant être réglé dans l'ouverture de passage (5) dans la direction axiale, et
- un dispositif à ailettes (29) avec un corps de retenue (42) de forme tubulaire et des ailes (43) s'étendant des deux côtés du corps de retenue (42) étant également prévu, les ailes (43) définissant un côté d'appui (44) pouvant être tourné vers un patient, et une unité de couplage (45) étant agencée ou formée entre le corps de retenue (42) et le corps de base (2) et le dispositif à ailettes (29) étant maintenu au moyen de l'unité de couplage (45) dans une position de couplage sur le corps de base (2),
**caractérisé en ce**
- **qu'**une fenêtre de visualisation (46) est formée dans le corps de retenue (42) de forme tubulaire, laquelle fenêtre de visualisation (46) est agencée sur un côté opposé au côté d'appui (44) et que, dans la position de couplage, un appendice (47) s'écartant dans la direction radiale du corps de base (2) fait saillie dans la fenêtre de visualisation (46),
- le corps de base (2) et le support de canule (6) étant constitués d'un matériau translucide ou transparent, généralement un matériau plastique.

2. Ensemble de canules (1) selon la revendication 1, **caractérisé en ce que** les sections de logement (38, 39, 40) sont chacune conçues avec une section transversale cylindrique creuse.

3. Ensemble de canules (1) selon la revendication 1 ou 2, **caractérisé en ce que** plusieurs, en particulier trois, sections de logement (38, 39, 40) sont prévues, formées les unes derrière les autres dans la direction axiale.

4. Ensemble de canules (1) selon l'une des revendications précédentes, **caractérisé en ce que** chacune des sections de logement (38, 39, 40) forme à son extrémité proximale une butée axiale pour l'extrémité proximale (12) de la canule (10) respective qui y est logée.

5. Ensemble de canules (1) selon la revendication 4, **caractérisé en ce que** l'extrémité proximale (12) respective de la canule (10) est appuyée contre la butée axiale correspondante de la section de logement respective (38, 39, 40) dans la direction de l'extrémité proximale (8) du support de canule (6).

6. Ensemble de canules (1) selon l'une des revendications précédentes, **caractérisé en ce que** le support de canule (6) est formé par une pièce de raccordement Luer femelle ou un composant d'un ensemble de canules de sécurité.

7. Ensemble de canules (1) selon l'une des revendications précédentes, **caractérisé en ce qu'**il est en outre prévu un élément de réglage (16) à action automatique, lequel élément de réglage (16) est agencé de manière à agir entre le corps de base (2) et le support de canule (6), et l'élément de réglage (16) déplace le support de canule (6) avec la canule (10) maintenue sur celui-ci d'une première position, dans laquelle la canule (10) dépasse de l'extrémité distale (3) du corps de base (2), vers une deuxième position, dans laquelle au moins l'extrémité distale (11) de la canule (10) est recouverte par le corps de base (2).

8. Ensemble de canules (1) selon l'une des revendications précédentes, **caractérisé en ce qu'**il est en outre prévu un dispositif de retenue (20) comportant plusieurs premiers éléments de retenue (21) et plusieurs deuxièmes éléments de retenue (22), lequel dispositif de retenue (20), lorsque les premiers et deuxième éléments de retenue sont en position d'engagement mutuel (21, 22), détermine la première position du support de canule (6) par rapport au corps de base (2), et **en ce que** les premiers éléments de retenue (21) sont agencés dans la zone de l'extrémité proximale (4) du corps de base (2) et les deuxièmes éléments de retenue (22) dans la zone de l'extrémité proximale (8) du support de canule (6).

9. Ensemble de canules (1) selon l'une des revendications précédentes, **caractérisé en ce qu'**il comprend en outre un dispositif de blocage (26) avec plusieurs premiers et deuxièmes éléments de blocage (27, 28), lequel dispositif de blocage (26), lorsque les premiers et deuxièmes éléments de blocage (27, 28) sont en engagement mutuel, détermine la deuxième position du support de canule (6) par rapport au corps de base (2), et **en ce que** les premiers éléments de blocage (27) sont agencés dans la zone de l'extrémité proximale (4) du corps de base (2) et les deuxièmes éléments de blocage (28) dans la zone de l'extrémité distale (7) du support de canule (6).

10. Ensemble de canules (1) selon l'une des revendications précédentes, **caractérisé en ce qu'**au moins un renfoncement de décharge (41) est agencé ou formé dans le support de canule (6) et **en ce que**, lorsque le support de canule (6) se trouve dans la première position, les premiers éléments de blocage (27) font saillie dans leur position non déformée dans l'au moins un renfoncement de décharge (41).

11. Ensemble de canules (1) selon l'une des revendications précédentes, **caractérisé en ce que** l'unité de couplage (45) comprend en outre un dispositif de sécurité anti-rotation en Y (48) avec au moins un premier élément de sécurité anti-rotation (49) sur ou dans le corps de base (2) et au moins un deuxième élément de sécurité anti-rotation (50) coopérant avec celui-ci dans ou sur le corps de retenue (42).

12. Ensemble de canules (1) selon la revendication 11, **caractérisé en ce que** l'au moins un premier élément de sécurité anti-rotation (49) est conçu comme une nervure et l'au moins un deuxième élément de sécurité anti-rotation (50) est conçu comme une rainure, et **en ce que** les éléments de sécurité anti-rotation (49, 50) présentent une orientation longitudinale parallèle par rapport à l'axe longitudinal (14).

13. Ensemble de canules (1) selon l'une des revendications précédentes, **caractérisé en ce que** l'unité de couplage (45) comprend en outre un dispositif de sécurité axiale (51) avec au moins un premier élément de sécurité axiale (52) sur ou dans le corps de base (2) et au moins un deuxième élément de sécurité axiale (53) coopérant avec celui-ci dans ou sur le corps de retenue (42).

14. Ensemble de canules (1) selon la revendication 13, **caractérisé en ce que** les éléments de sécurité axiaux (52, 53) sont réalisés, vus en coupe axiale, avec une section transversale approximativement triangulaire, une première ligne de délimitation (54) de la section transversale triangulaire définissant, dans la direction axiale de déplacement du dispositif à ailettes (29) sur le corps de base (2), une rampe ascendante sur le côté opposé à l'axe longitudinal (14), et **en ce qu'**une deuxième ligne de délimitation (55) de la section transversale triangulaire est orientée dans un plan normal par rapport à l'axe longitudinal (14).

15. Ensemble de canules (1) selon l'une des revendications précédentes, **caractérisé en ce qu'**il est en outre prévu une gaine de protection (30) de forme notamment tubulaire, et **en ce que** la gaine de protection (30) est agencée à l'extrémité distale (3) du corps de base (2) et est maintenue de manière amovible sur celui-ci, la canule saillante (10) étant recouverte par la gaine de protection (30) dans la première position du corps de base (2) dans la direction distale.

16. Ensemble de canules (1) selon la revendication 15, **caractérisé en ce qu'**au moins deux premières nervures longitudinales (57) et au moins deux deuxièmes nervures longitudinales (58) sont agencées ou formées sur une surface intérieure (56) de la gaine de protection (30) tournée vers l'axe longitudinal (14), réparties sur la circonférence, et **en ce qu'**une première nervure longitudinale (57) et une deuxième nervure longitudinale (58) sont agencées en alternance dans la direction circonférentielle.

17. Ensemble de canules (1) selon la revendication 16, **caractérisé en ce que**, lorsque la section transversale de la gaine de protection (30) n'est pas déformée, les premières extrémités (59) des premières nervures longitudinales (57) se terminent à une première distance radiale (60) devant l'axe longitudinal (14) et les deuxièmes extrémités (61) des deuxièmes nervures longitudinales (58) se terminent à une deuxième distance radiale (62) devant l'axe longitudinal (14), la première distance radiale (60) étant supérieure à la deuxième distance radiale (62).

18. Ensemble de canules (1) selon la revendication 17, **caractérisé en ce que** les premières extrémités (59) des premières nervures longitudinales (57) sont agencées sur une première trajectoire circulaire et les deuxièmes extrémités (61) des deuxièmes nervures longitudinales (58) sont agencées sur une deuxième trajectoire circulaire, et les deux trajectoires circulaires sont agencées de manière concentrique par rapport à l'axe longitudinal (14).

19. Ensemble de canules (1) selon la revendication 17 ou 18, **caractérisé en ce qu'**une valeur différentielle de la première distance radiale (60) moins la deuxième distance radiale (62) provient d'une plage de valeurs différentielles dont la limite inférieure est de 0,01 mm, de préférence de 0,04 mm, et dont la limite supérieure est de 0,1 mm, de préférence 0,06 mm.

20. Ensemble de canules (1) selon l'une des revendications 16 à 19, **caractérisé en ce que** le nombre total de premières nervures longitudinales (57) et de deuxièmes nervures longitudinales (58) est un nombre pair et est choisi parmi un nombre total de quatre, six, huit ou dix.

21. Ensemble de canules (1) selon l'une des revendications 16 à 20, **caractérisé en ce que** les premières nervures longitudinales (57) et/ou les deuxièmes nervures longitudinales (58) présentent chacune une forme ondulée en coupe radiale.
